# EUROPEAN PATENT SPECIFICATION

(11) **EP 1 592 405 B1**
(45) Date of publication and mention of the grant of the patent: **28.05.2008**
(21) Application number: 04710431.0
(22) Date of filing: 12.02.2004
(51) Int. Cl.: A61K 9/16, A61K 31/704

(54) **COMPOSITION FOR CHEMOEMBOLOTHERAPY OF SOLID TUMORS**
ZUSAMMENSETZUNGEN FÜR DIE CHEMOEMBOLOTHERAPIE SOLIDER TUMOREN
COMPOSITION UTILISEE DANS LA CHIMIO-EMBOLOTHERAPIE DE TUMEURS SOLIDES

(30) Priority: 12.02.2003 EP 03250868; 07.03.2003 GB 0305322
(43) Date of publication of application: 09.11.2005
(73) Proprietor: Biocompatibles UK Limited, Farnham Surrey GU9 8QL (GB)
(72) Inventor: LEWIS, Andrew, Lennard, Biocompatibles UK Limited, Farnham, Surrey GU9 8QL (GB); STRAFORD, Peter, W., Biocompatibles UK Limited, Farnham, Surrey GU9 8QL (GB); LEPPARD, Simon, William, Biocompatibles UK Limited, Farnham, Surrey GU9 8QL (GB); HALL, Brenda, Biocompatibles UK Limited, Farnham, Surrey GU9 8QL (GB); GONZALEZ FAJARDO, Maria, V., Biocompatibles UK Ltd, Farnham, Surrey GU9 8QL (GB); GARCIA, Pedro, Biocompatibles UK Limited, Farnham, Surrey GU9 8QL (GB)
(74) Representative: Jones, Helen M.M.
(86) International application number: PCT/GB2004/000548
(87) International publication number: WO 2004/071495

(56) References cited:
- WO-A-01/68720
- WO-A-95/13798
- WO-A-99/12577
- DATABASE WPI Section Ch, Week 199507 Derwent Publications Ltd., London, GB; Class B04, AN 1995-048756 XP002246806 & JP 06 329542 A (KIBUN FOOD CHEMIPHAR KK) 29 November 1994 (1994-11-29)

## Description

The present invention relates to compositions a polymeric embolic material and a therapeutic agent incorporated into the polymer matrix. The composition is of use for embolising tumours and delivering cytotoxic agents thereto.

Embolotherapy is a growing area of interventional medicine but normally relies upon the transarterial approach of the catheter to a desired location whereupon an agent is released in order to occlude a particular vessel. This treatment has been used in order to block the blood supply to certain hypervascularised tumours such as hepatocellular carcinoma and more recently is becoming a popular choice of treatment for uterine fibroids.

There is a range of embolic materials in clinical use, that require transcatheter delivery to the site of embolisation, whereupon they are released into the blood stream to block it. This is achieved either by a physical blocking of the vessel using small particles or spheres, or in the case of liquid embolic agents, require some sort of phase change or reaction to set the flowable material and form a cast within the vessel.

The most popular particulate-based embolic agent is poly(vinyl alcohol) (PVA) foam particles (e.g. Ivalon) which has been used for several decades. Recently, this material has been available in particulate, rather than sheet form, and does not require granulation by the surgeon prior to delivery.

In WO-A-0168720, PVA based compositions for embolotherapy are described. The PVA is, initially, derivatised to form a macromonomer, having pendant acrylic groups. Subsequently, these acrylic groups are polymerised, optionally in the presence of comonomer, to form a water-insoluble water-swellable polymer matrix. The polymerisation reaction may be carried out *in situ,* whereby the PVA is rendered water-insoluble after delivery into the vessel, at the embolisation site. Alternatively, the polymerisation is conducted prior to delivery, generally to form microspheres, which are delivered in suspension in an aqueous vehicle.

In WO-A-0168720, it is suggested that biologically active agents may be included in the embolic compositions, whereby active agent may be delivered from the formed hydrogel. One class of active agents is chemo therapeutic agents. Examples of chemo therapeutic agents are cisplatin, doxorubicin and mitomycin. Some general guidance is given as to methods of incorporating the active agents into the embolic compositions. Where the composition is a liquid which is cured *in situ,* the active may be simply mixed with the liquid. Where the articles are preformed, it is suggested that the active may be incorporated by "encapsulation", or by coating onto the surface. There are no worked examples in which a therapeutic agent is incorporated into any type of composition.

Microspheres of hydrogel material formed from poly(hydroxyethyl methacrylate), hydrolysed poly(methyl methacrylate) and PVA crosslinked using aldehyde crosslinking agents such as glutaraldehyde, have also been used as embolic agents. Hydroxyethyl methacrylate may be copolymerised with comonomers, for instance having acidic groups. For instance, a crosslinked copolymer of hydroxyethyl methacrylate with about 1-2 mole% acrylic acid cross-linked by 0.3-1.0mole% ethylene glycol dimethacrylate, has an equilibrium water content in the range 55-60% by weight, and has been used as a contact lens formulation for many years.

One embolic product on the market is marketed by Biosphere, which comprises microspheres of trisacrylgelatin having a coating of collagen. Collagen has an overall cationic charge as physiological pH's. In Ball, D.S. et al., J. Vasc. Interv. Radiol. (2003), 14, 83-88, Biosphere show that the microspheres' mechanical characteristics are not adversely affected when admixed with a range of drugs commonly administered along with the embolic compositions. Doxorubicin, cisplatin and mitoxantrone are specifically tested.

Doxorubicin and other anthracyclines have been incorporated into a variety of polymeric matrices based delivery systems, such as microspheres of polylactides or polyglycolides and cross-linked fibrinogen and albumin microspheres. In WO95/13798 microspheres of cross-linked dextrasulphate and albumin having sizes 2.0-63 µm are used to deliver doxorubicin, for instance for embolotherapy of solid tumours. Juni, K. et al in Chem. Pharm. Bull. (1985), 33(1), 313-318 describe the incorporation of doxorubicin into poly(lactic acid) microspheres and the delivery of the composition intra arterially to dog liver. The composition embolised peripheral hepatic arteries. These types of microspheres are hard and are not easy to store and deliver. Doxorubicin has been covalently linked to the surface of cross-linked poly(vinyl alcohol) and tested for its cytotoxic properties (Wingard, L B et al. Cancer Research (1985) 45(8) 3529-3536). Since the drug is covalently bonded to the polymer it must be cleaved before being released from the surface and hence may not be released under physiological conditions.

In JP-A-06-329542 (1995) particles of ionically cross-linked alginic acid containing anti-cancer agents such as adriamycin are used in embolotherapy of tumours.

Jones, C. et al in Brit. J. Cancer (1989) 59(5) describe incorporation of doxorubicin into ion-exchange microspheres and the use of the compositions in the chemoembolotherapy of tumours in a rat model.

A new composition according to the invention suitable for embolisation, comprises particles having a matrix of water-swellable water-insoluble polymer and, absorbed in the matrix, a water-soluble therapeutic agent, and is characterised in that the polymer has an overall anionic charge at a pH in the range 6 to 8 in the range 0.1 to 10 meq/g and comprises covalently cross-linked poly(vinyl alcohol), in that the particles, when swollen to equilibrium in water have particle sizes in the range 40-1500 µm and in that the therapeutic agent is an anthracycline compound having at least one amine group.

The polymer in the invention must be water-swellable, but water-insoluble. In the presence of aqueous liquid, therefore, the polymer will form a hydrogel. The polymer is covalently crosslinked, although it may be appropriate for the polymer to be ionically crosslinked, at least in part. The polymer may be formed by polymerising ethylenically unsaturated monomers in the presence of di- or higher-functional crosslinking monomers, the ethylenically unsaturated monomers including an anionic monomer.

Another type of polymer which may be used to form the water-swellable water-insoluble matrix is polyvinyl alcohol crosslinked using aldehyde type crosslinking agents such as glutaraldehyde. For such products, the polyvinyl alcohol must be rendered anionic, for instance by providing pendant anionic groups by reacting a functional acidic group containing monomer with the hydroxyl groups. Examples of suitable reagents are di-acids, for instance dicarboxylic acids.

The invention is of particular value where the polymer matrix is formed of a polyvinyl alcohol macromer, having more than one ethylenically unsaturated pendant group per molecule, by copolymerisation with ethylenically unsaturated monomers including an acidic monomer. The PVA macromer may be formed, for instance, by providing PVA polymer, of a suitable molecular weight such as in the range 1000 to 500,000 D, preferably 10,000 to 100,000 D, with pendant vinylic or acrylic groups. Pendant acrylic groups may be provided, for instance, by reacting acrylic or methacrylic acid with PVA to form ester linkages through some of the hydroxyl groups. Methods for attaching vinylic groups capable of polymerisation onto polyvinyl alcohol are described in, for instance, US 4,978,713 and, preferably, US 5,508,317 and 5,583,163. Thus the preferred macromer comprises a backbone of polyvinyl alcohol to which is linked, via a cyclic acetal linkage, to an (alk)acrylaminoalkyl moiety. Example 1 describes the synthesis of such a macromer. Preferably the PVA macromers have about 2 to 20 pendant ethylenic groups per molecule, for instance 5 to 10.

Where PVA macromers are copolymerised with ethylenically unsaturated monomers including an acidic monomer, the acidic monomer preferably has the general formula I

Y¹BQ

in which Y¹ is selected from 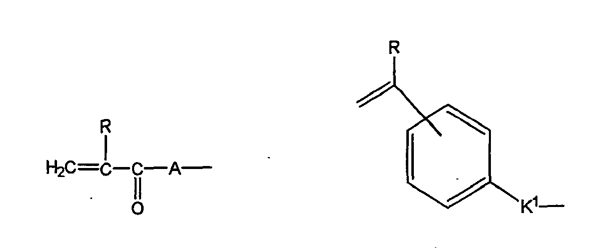 CH₂=C(R)-CH₂-O-, CH₂=C(R)-CH₂ OC(O)-, CH₂=C(R)OC(O)-, CH₂=C(R)-O-, CH₂=C(R)CH₂OC(O)N(R¹)-, R²OOCCR=CRC(O)-O-, RCH=CHC(O)O-, RCH=C(COOR²)CH₂-C(O)-O-, 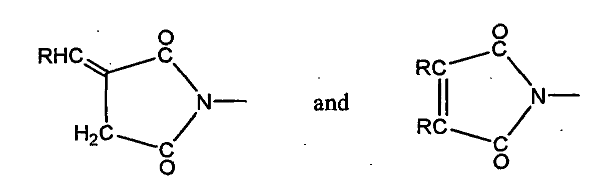 wherein:
R is hydrogen or a C₁-C₄ alkyl group;
R¹ is hydrogen or a C₁-C₄ alkyl group;
R² is hydrogen or a C₁₋₄ alkyl group or BQ where B and Q are as defined below;
A is -O- or -NR¹-;
K¹ is a group -(CH₂)ᵣOC(O)-, -(CH₂)ᵣC(O)O-, -(CH₂)ᵣOC(O)O-, -(CH₂)ᵣNR³-, -(CH₂)ᵣNR³C(O)-, -(CH₂)ᵣC(O)NR³-, -(CH₂)ᵣNR³C(O)O-, -(CH₂)ᵣOC(O)NR³-, -(CH₂)ᵣNR³C(O)NR³- (in which the groups R³ are the same or different), -(CH₂)ᵣO-, -(CH₂)ᵣSO₃-, or, optionally in combination with B¹, a valence bond and r is from 1 to 12 and R³ is hydrogen or a C₁-C₄ alkyl group;
B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if Q or Y¹ contains a terminal carbon atom bonded to B a valence bond; and
Q is an anionic group.

The anionic group may be, for instance, a carboxylate, carbonate, sulphonate, sulphate, nitrate, phosphonate or phosphate group, preferably a sulphonate group. The monomer may be polymerised as the free acid or in salt form. Preferably the pKₐ of the conjugate acid is less than 5.

In the monomer of general formula I preferably Y¹ is a group CH₂=CRCOA- in which R is H or methyl, preferably methyl, and in which A is preferably NH. B is preferably an alkanediyl group of 1 to 12, preferably 2 to 6 carbon atoms.

One particularly preferred type of monomer is an (alk)acrylamido alkane-sulphonic acid, such as 2-acrylamido-2-methyl-1-propane-sulphonic acid (AMPS).

There may be included in the ethylenically unsaturated monomer diluent monomer, for instance non-ionic monomer. Such monomer may be useful to control the pKₐ of the acid groups, to control the hydrophilicity or hydrophobicity of the product, to provide hydrophobic regions in the polymer, or merely to act as inert diluent. Examples of non-ionic diluent monomer are, for instance, alkyl (alk) acrylates and (alk) acrylamides, especially such compounds having alkyl groups with 1 to 12 carbon atoms, hydroxy, and dihydroxy-substituted alkyl(alk) acrylates and -(alk) acrylamides, vinyl lactams, styrene and other aromatic monomers.

The ethylenically unsaturated monomer may also include zwitterionic monomer, for instance to increase the hydrophilicity, lubricity, biocompatibility and/or haemocompatibility of the particles. Suitable zwitterionic monomers are described in our earlier publications WO-A-9207885, WO-A-9416748, WO-A-9416749 and WO-A-9520407. Preferably a zwitterionic monomer is 2-methacryloyloxy-2'-trimethylammonium ethyl phosphate inner salt (MPC).

In the polymer matrix, the level of anion is in the range 0.1 to 10 meq g⁻¹, preferably at least 1.0 meq g⁻¹.

Where PVA macromer is copolymerised with other ethylenically unsaturated monomers, the weight ratio of PVA macromer to other monomer is preferably in the range of 50:1 to 1:5, more preferably in the range 20:1 to 1:2. In the ethylenically unsaturated monomer the anionic monomer is preferably present in an amount in the range 10 to 100 mole%, preferably at least 25 mole%.

Preferably the water-insoluble water-swellable polymer has an equilibrium water content measured by gravimetric analysis of 40 to 99 weight %, preferably 75 to 95%.

The polymer may be formed into particles in several ways. For instance, the crosslinked polymer may be made as a bulk material, for instance in the form of a sheet or a block, and subsequently be comminuted to the desired size. Alternatively, the crosslinked polymer may be formed as such in particulate form, for instance by polymerising in droplets of monomer in a dispersed phase in a continuous immiscible carrier. Examples of suitable water-in-oil polymerisations to produce particles having the desired size, when swollen, are known. For instance US 4,224,427 describes processes for forming uniform spherical beads of up to 5 mm in diameter, by dispersing water-soluble monomers into a continuous solvent phase, in a presence of suspending agents. Stabilisers and surfactants may be present to provide control over the size of the dispersed phase particles. After polymerisation, the crosslinked microspheres are recovered by known means, and washed and optionally sterilised. Preferably the particles eg microspheres, are swollen in an aqueous liquid, and classified according to their size.

The therapeutic active used in the present invention is an anthracycline compound, which comprises an anthraquinone group to which is attached an amine sugar. The amino group on the sugar is believed to associate with the anionic groups in the polymer matrix, to allow high levels of loading and controlled delivery after administration.

Examples of suitable anthracyclines have the general formula II

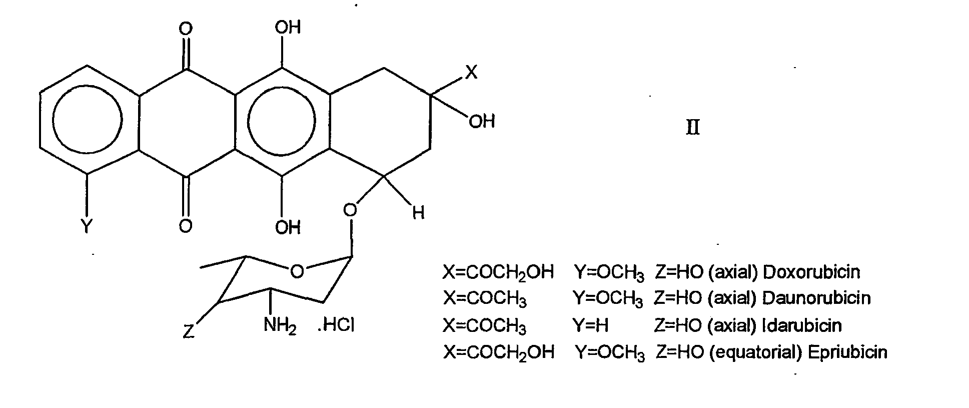

We have found that doxorubicin, which has been thoroughly tested for efficacy on various tumours, has particularly interesting loading and release characteristics. The drug appears to have a particular affinity for poly(vinyl alcohol-graft-acrylamido propane sulphonic acid), so that high levels of doxorubicin are capable of incorporation into the polymer, and release over many days.

In the invention it is important that the drug is not covalently attached to the polymer matrix.

The therapeutic active may be incorporated into the polymer matrix by a variety of techniques. In one method, the therapeutic active may be mixed with a precursor of the polymer, for instance a monomer or macromer mixture or a cross-linkable polymer and cross-linker mixture, prior to polymerising or crosslinking. Alternatively, the active may be loaded into the polymer after it has been crosslinked. For instance, particulate dried polymer may be swollen in a solution of therapeutic active, preferably in water, optionally with subsequent removal of non-absorbed agent and/or evaporation of solvent. A solution of the active, in an organic solvent such as an alcohol, or, more preferably, in water, may be sprayed onto a moving bed of particles, whereby drug is absorbed into the body of the particles with simultaneous solvent removal. Most conveniently, we have found that it is possible merely to contact swollen particles suspended in a continuous liquid vehicle, such as water, with a solution of drug, over an extended period, whereby drug becomes absorbed into the body of the particles. This is believed to be analogous to a cation exchange type process. The swelling vehicle may subsequently be removed or, conveniently, may be retained with the particles as part of the product for subsequent use as an embolic agent.

In one particularly preferred embodiment the swollen particles are separated from swelling vehicle not absorbed into the matrix by a simple gel/liquid separation technique such as by filtration through a filter having suitable apertures, conveniently a glass filter. The slurry of swollen particles with little or no extra-particulate liquid may be pumped into suitable storage containers for sterilisation and storage as it is. It is found that the slurry is sufficiently stable, in that little exudation of liquid nor loss of drug occurs during storage in such a form.

Alternatively, the suspension of particles can be filtered to remove any remaining drug loading solution and the particles dried by any of the classical techniques employed to dry pharmaceutical -based products. This could include, but is not limited to, air drying at room or elevated temperatures or under reduced pressure or vacuum; classical freeze-drying; atmospheric pressure-freeze drying; solution enhanced dispersion of supercritical fluids (SEDS). Alternatively the drug-loaded microspheres may be dehydrated using an organic solvent to replace water in a series of steps, followed by evaporation of the more volatile organic solvent. A solvent should be selected which is a non solvent for the drug.

In brief, a typical classical freeze drying process might proceed as follows: the sample is aliquoted into partially stoppered glass vials, which are placed on a cooled, temperature controlled shelf within the freeze dryer. The shelf temperature is reduced and the sample is frozen to a uniform, defined temperature. After complete freezing, the pressure in the dryer is lowered to a defined pressure to initiate primary drying. During the primary drying, water vapour is progressively removed from the frozen mass by sublimation whilst the shelf temperature is controlled at a constant, low temperature. Secondary drying is initiated by increasing the shelf temperature and reducing the chamber pressure further so that water absorbed to the semi-dried mass can be removed until the residual water content decreases to the desired level. The vials can be sealed, *in situ,* under a protective atmosphere if required.

Atmospheric pressure freeze drying is accomplished by rapidly circulating very dry air over a frozen product. In comparison with the classical freeze-drying process, freeze-drying without a vacuum has a number of advantages. The circulating dry gas provides improved heat and mass transfer from the frozen sample, in the same way as washing dries quicker on a windy day. Most work in this area is concerned with food production, and it has been observed that there is an increased retention of volatile aromatic compounds, the potential benefits of this to the drying of biologicals is yet to be determined. Of particular interest is the fact that by using atmospheric spray drying processes instead of a cake, a fine, free-flowing powder is obtained. Particles can be obtained which have submicron diameters, this is tenfold smaller than can be generally obtained by milling. The particulate nature, with its high surface area results in an easily rehydratable product, currently the fine control over particle size required for inhalable and transdermal applications is not possible, however there is potential in this area.

The composition which is administered to a patient in need of embolotherapy having a solid tumour, for instance a hepatocellular carcinoma, is an aqueous suspension of swollen particles containing absorbed drug. It is often desirable for the suspension to be mixed prior to delivery with an imaging agent such as a conventional radiopaque agent, as is used for gel type embolic compositions. For example an aqueous suspension of swollen particles containing absorbed drug may be mixed immediately prior to administration with a liquid radiopaque agent conventionally used with embolic agents, e.g. lipiodol, in amounts in the range 2:1 to 1:2, preferably about 1:1 by volume. For the embodiment of the invention comprising a slurry of swollen particles with absorbed drug but with little or no extra-particulate liquid, the slurry and contrast (radiopaque) agent may, similarly, be mixed together immediately before delivery, for instance in amounts in the range 1:5 to 2:1, preferably in the range 1:2 to 1:1 by volume. Where the compositions containing drug are supplied for use in dried form the particles may be added dry to contrast agent, or preferably, are initially swollen in an aqueous vehicle such as physiological saline, to form a slurry or suspension, before being blended with contrast agent prior to delivery. Alternatively or additionally the particles may be pre-loaded with radiopaque material, in addition to the anthracycline. The composition which is administered may also be admixed with other therapeutic agents, or may be administered in separately but in combination with other therapeutic agents. Usually the composition is administered from a reservoir in a syringe using the conventional delivery devices, such as an intra-arterial catheter.

The embolic composition as administered to the patient in need of embolisation therapy, may be delivered as a single one-off dosage. Embolisation is monitored by following the contrast agent using conventional techniques. It may be found to be desirable for a second dose of an embolic composition, preferably for a second dose of an embolic composition, preferably the chemoembolic composition useful in the invention, to be delivered at a time interval after the first dose, for instance, to embolise newly formed blood vessels supplying the tumour e.g. after 4 to 10 weeks from the first treatment for a doxorubicin - containing composition. The composition will be administered in a drug dosage in the range 25-100 mg/m² per treatment, although it may be possible to use higher dosages following adequate safety assessments. Preferred dosages per treatment for doxorubicin may be above 50 mg/m², for instance up to 100 mg/m² or more. It is generally believed to be undesirable to administer more than 150 mg per patient per treatment.

There is provided as a preferred embodiment of the invention the use of an anthracycline compound in the manufacture of a composition for use in the treatment of a solid tumour by embolotherapy, in which treatment the anthracycline is delivered from a polymer matrix formed by the copolymerisation of a poly(vinyl alcohol) macromer having at least 2 pendant ethylenically unsaturated groups per molecule and an ethylenically unsaturated anionic monomer.

The polymer matrix is preformed before administration, as described above.

The present invention is illustrated in the following examples for which the results are shown in the figures as follows:
Figure 1 shows the results of example 2;
Figure 2 shows the results of example 3;
Figure 3 shows the results of example 4;
Figure 4 shows the results of example 5;
Figure 5 shows the results of example 6;
Figures 6a and b show the results of example 7;
Figure 7 shows the results of example 10;
Figure 8 shows the results of example 12;
Figure 9 shows the results of example 13;
Figures 10 and 11 show the results of example 14;
Figure 12 shows the results of example 15;
Figure 13 shows the results of example 16;
Figure 14 shows the results of example 15;
Figure 15 shows the results of example 18;
Figure 16 shows the results of example 19;
Figure 17 shows the results of example 23;
Figure 18 shows the results of example 25;
Figure 19 shows the results of example 26; and
Figures 20 to 22 show the results of Example 27.

### Example 1: Outline Method for the Preparation of Microspheres

### Nelfilcon B macromer synthesis:

The first stage of microsphere synthesis involves the preparation of Nelfilcon B - a polymerisable macromer from the widely used water soluble polymer PVA. Mowiol 8-88 poly(vinyl alcohol) (PVA) powder (88% hydrolised, 12% acetate content, average molecular weight about 67,000D) (150g) (Clariant, Charlotte, NC USA) is added to a 2litre glass reaction vessel. With gentle stirring, 1000ml water is added and the stirring increased to 400rpm. To ensure complete dissolution of the PVA, the temperature is raised to 99 ±9°C for 2-3 hours. On cooling to room temperature N-acryloylaminoacetaldehyde (NAAADA) (Ciba Vision, Germany) (2.49g or 0.104mmol/g of PVA) is mixed in to the PVA solution followed by the addition of concentrated hydrochloric acid (100ml) which catalyses the addition of the NAAADA to the PVA by transesterification. The reaction proceeds at room temperature for 6-7 hours then stopped by neutralisation to pH 7.4 using 2.5M sodium hydroxide solution. The resulting sodium chloride plus any unreacted NAAADA is removed by diafiltration (step 2).

### Diafiltration of macromer:

Diafiltration (tangential flow filtration) works by continuously circulating a feed solution to be purified (in this case nelfilcon B solution) across the surface of a membrane allowing the permeation of unwanted material (NaCl, NAAADA) which goes to waste whilst having a pore size small enough to prevent the passage of the retentate which remains in circulation.

Nelfilcon B diafiltration is performed using a stainless steel Pellicon 2 Mini holder stacked with 0.1m² cellulose membranes having a pore size with a molecular weight cut off of 3000 (Millipore Corporation, Bedford, MA USA). Mowiol 8-88 has a weight average molecular weight of 67000 and therefore has limited ability to permeate through the membranes.

The flask containing the macromer is furnished with a magnetic stirrer bar and placed on a stirrer plate. The solution is fed in to the diafiltration assembly via a Masterflex LS peristaltic pump fitted with an Easy Load If pump head and using LS24 class VI tubing. The Nelfilcon is circulated over the membranes at approximately 340 kPa (50psi) to accelerate permeation. When the solution has been concentrated to about 1000ml the volume is kept constant by the addition of water at the same rate that the filtrate is being collected to waste until 6000ml extra has been added. Once achieved, the solution is concentrated to 20-23% solids with a viscosity of 1.7-3.4 Pa-s (1700-3400 cP) at 25°C. Nelfilcon is characterised by GFC, NMR and viscosity.

### Microsphere Synthesis:

The spheres are synthesised by a method of suspension polymerisation in which an aqueous phase (nelfilcon B) is added to an organic phase (butyl acetate) where the phases are immiscible. By employing rapid mixing the aqueous phase can be dispersed to form droplets, the size and stability of which can be controlled by factors such as stirring rates, viscosity, ratio of aqueous/organic phase and the use of stabilisers and surfactants which influence the interfacial energy between the phases. Two series of microspheres are manufactured, a low AMPS and a higher AMPS series, the formulation of which are shown below.

| | |
|---|---|
| A | High AMPS: |
| Aqueous: | ca 21% w/w Nelfilcon B solution (400 ±50g approx) |
| | ca 50% w/w 2-acrylamido-2-methylpropanesulphonate Na salt |
| | (140±10g) |
| | Purified water (137±30g) |
| | Potassium persulphate (5.22±0.1g) |
| | Tetramethyl ethylene diamine TMEDA (6.4±0.1ml) |
| Organic: | n-Butyl acetate (2.7 ±0.3L) |
| | 10% w/w cellulose acetate butyrate in ethyl acetate (46±0.5g) |
| | (stabiliser) |
| | Purified water (19.0 ±0.5ml) |
| B | Low AMPS: |
| Aqueous: | ca 21 % w/w Nelfilcon B solution (900 ±100g approx) |
| | ca 50% w/w 2-acryamido-2-methylpropanesulphonate Na salt |
| | (30.6 ±6g) |
| | Purified water (426±80g) |
| | Potassium persulphate (20.88±0.2g) |
| | TMEDA (25.6±0.5ml) |
| Organic: | n-Butyl acetate (2.2 ±0.3L) |
| | 10% w/w cellulose acetate butyrate (CAB) in ethyl acetate |
| | (92±1.0g) |
| | Purified water (16.7 ±0.5ml) |

A jacketed 4000ml reaction vessel is heated using a computer controlled bath (Julabo PN 9-300-650) with feedback sensors continually monitoring the reaction temperature.

The butyl acetate is added to the reactor at 25°C followed by the CAB solution and water. The system is purged with nitrogen for 15 minutes before the PVA macromer is added. Cross linking of the dispersed PVA solution is initiated by the addition of TMEDA and raising the temperature to 55°C for three hours under nitrogen. Crosslinking occurs via a redox initiated polymerisation whereby the amino groups of the TMEDA react with the peroxide group of the potassium persulphate to generate radical species. These radicals then initiate polymerisation and crosslinking of the double bonds on the PVA and AMPS transforming the dispersed PVA-AMPS droplets into insoluble polymer microspheres. After cooling to 25°C the product is transferred to a filter reactor for purification where the butyl acetate is removed by filtration followed by:
- Wash with 2 x 300ml ethyl acetate to remove butyl acetate and CAB
- Equilibrate in ethyl acetate for 30mins then filtered
- Wash with 2 x 300 ml ethyl acetate under vacuum filtration
- Equilibrate in acetone for 30mins and filter to remove ethyl acetate, CAB and water
- Wash with 2 x 300ml acetone under vacuum filtration
- Equilibrate in acetone overnight
- Wash with 2 x 300ml acetone under vacuum
- Vacuum dry, 2hrs, 55°C to remove residual solvents.

### Dyeing:

This step is optional but generally unnecessary when drug is loaded with a coloured active (as this provides the colour). When hydrated the microsphere contains about 90% (w/w) water and can be difficult to visualise. To aid visualisation in a clinical setting the spheres are dyed blue using reactive blue #4 dye (RB4). RB4 is a water soluble chlorotriazine dye which under alkaline conditions will react with the pendant hydroxyl groups on the PVA backbone generating a covalent ether linkage. The reaction is carried out at pH12 (NaOH) whereby the generated HCl will be neutralised resulting in NaCl.

Prior to dyeing the spheres are fully re-hydrated and divided into 35g aliquots (treated individually). Dye solution is prepared by dissolving 0.8g RB4 in 2.5M NaOH solution (25ml) and water (15ml) then adding to the spheres in 2I of 80g/l⁻¹ saline. After mixing for 20mins the product is collected on a 32 µm sieve and rinsed to remove the bulk of the unreacted dye.

### Extraction:

An extensive extraction process is used to remove any unbound or non specifically adsorbed RB4. The protocol followed is as shown:
- Equilibrate in 2l water for 5mins. Collect on sieve and rinse.
   Repeat 5 times
- Equilibrate in 2I solution of 80mM disodium hydrogen phosphate in 0.29% (w/w) saline. Heat to boiling for 30mins. Cool, collect on sieve and wash with 1l saline. Repeat twice more.
- Collect, wash on sieve the equilibrate in 2l water for 10mins.
- Collect and dehydrate in 1l acetone for 30mins.
- Combine all aliquots and equilibrate overnight in 2l acetone.

### Sieving:

The manufactured microsphere product ranges in size from 100 to 1200 µm and must undergo fractionation through a sieving process using a range of mesh sizes to obtain the nominal distributions listed below.
1. 100 - 300µm
2. 300 - 500µm
3. 500 - 700µm
4. 700 - 900µm
5. 900 - 1200µm

Prior to sieving the spheres are vacuum dried to remove any solvent then equilibrated at 60°C in water to fully re-hydrate. The spheres are sieved using a 316L stainless steel vortisieve unit (MM Industries, Salem Ohio) with 38 cm (15") stainless steel sieving trays with mesh sizes ranging from 32 to 1000µm. Filtered saline is recirculated through the unit to aid fractionation. Spheres collected in the 32 µm sieve are discarded.

### Example 2: Loading of Doxorubicin

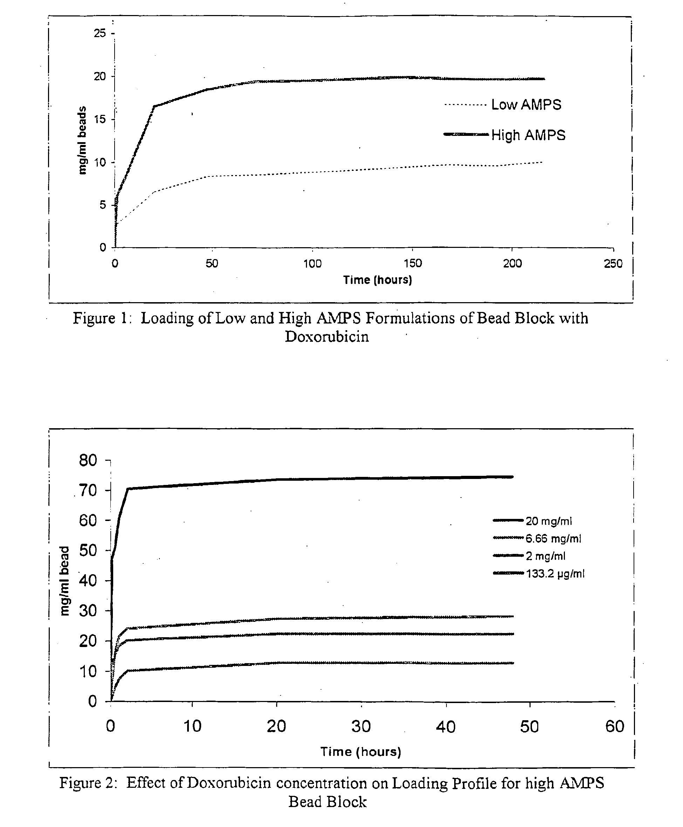

For this experiment the low AMPS microspheres prepared as in example 1 were used. For each size of bead used, 0.5 ml was transferred in to 2, 1 ml syringes, one for drug take up and the second to act as a control. The sizes chosen for the experiment were, 106 - 300 µm, 300 - 500 µm, 500 - 710 µm and 850 - 1000 µm. Additionally a further 3 syringes of the 500 - 710 µm were prepared in order to validate the procedure. 11, 10 ml glass vials were covered in foil, to prevent degradation of the doxorubicin by light for the duration of the experiment. A standard curve was created. Using the 80 ml, 20 mg/ml drug solution, the following concentrations were prepared and their absorbances (at 483nm) measured: 100 µg/ml, 50 µg/ml, 25 µg/ml, 12.5 µg/ml, 6.25 µg/ml and 3.125 µg/ml. The resulting absorbances were plotted on a graph and the equation of the line used to calculate the concentration of drug that was up-taken by the beads in the experiment. Four of the vials were filled with 5 ml of distilled water (ROMIL) to be used as controls when the beads were added. To the remaining 7 vials were added 5 ml of the drug solution at the desired concentration. The starting absorbance and therefore concentration of the solution was already known from the preparation of the standard curve. (In order to measure the absorbance of the 20 mg/ml solution it was necessary to dilute it 200 times, using the concentration 100 µg/ml. This 1:200 dilution was carried through for the duration of measuring the uptake of the solution by the beads.) The stopwatch was started as soon as the first set of microspheres were added to the first drug containing vial, microspheres were added to each of the remaining 6 vials working from smallest to largest. Once sealed using the caps they were placed on the rotary mixer. The process was repeated for the control samples. The absorbances were measured in the same order as the vials were set up at time intervals of 0.167 hr (10 min), 0.5 hr, 1 hr, 2 hr, 24 hr and 96 hr. From the data the amount of drug (in mg) per 1 ml of microspheres and the % uptake of drug by 1 ml of microspheres could be calculated. The results are shown in Figure 1.

### Example 3: Effect of Drug Concentration on Loading

Following the procedure outline in Example 2, it was possible to load a range of different concentrations of Doxorubicin into the high AMPS microsphere formulation. The majority of the drug was seen to load into the microspheres (500-710 µm size range) within a few hours (see Figure 2). The loading can be seen to be far higher than for the low AMPS formulation on a weight basis.

### Example 4: Effect of Microsphere Size on Loading

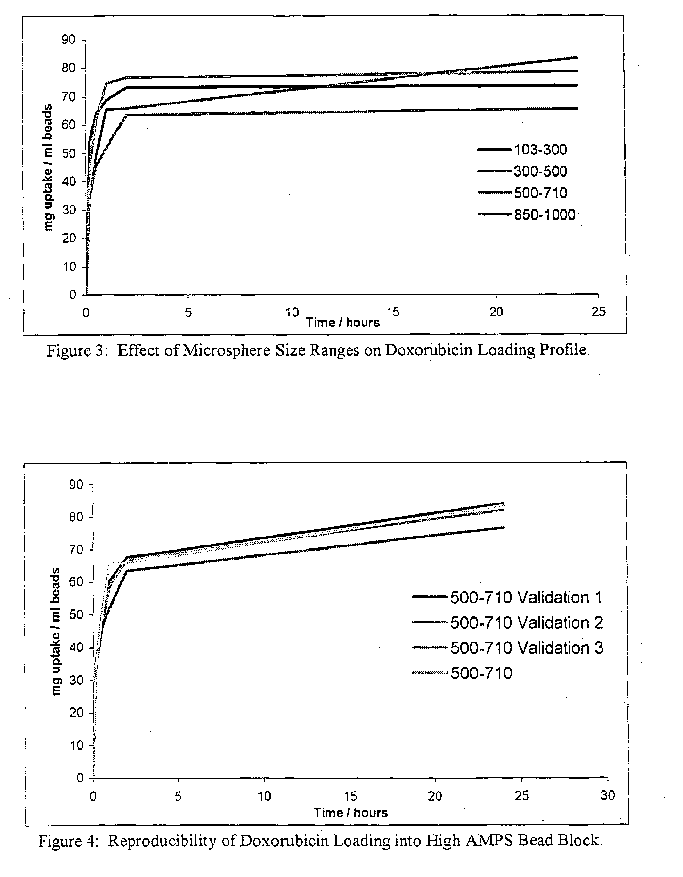

Loading of doxorubicin was conducted on several different size ranges of microspheres to enable comparison of the uptake. Whilst the smaller microspheres were seen to load drug more rapidly, continued loading over a 24 hour period suggests that an equivalent weight of microspheres will equilibrate to about the same drug loading. The more rapid uptake is attributed to increased surface area of the smaller microspheres (see fig 3).

### Example 5: Reproducibility of Loading

The loading experiments outlined in example 2 were repeated a number of times in order to measure the reproducibility in loading of the doxorubicin. High AMPS microspheres of 500-710 µm size range were loaded from a 20mg/ml drug solution in water and the drug uptake monitored over time (Figure 4).

### Example 6: Elution of Doxorubicin from Microspheres

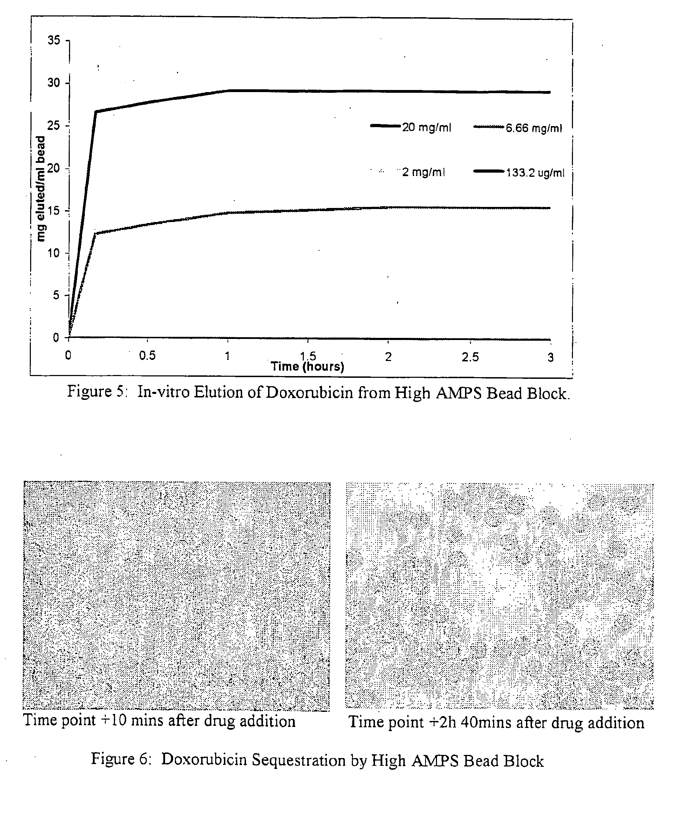

High AMPS microspheres were loaded with various concentrations of doxorubicin and the microspheres eluted into 250 ml of distilled water (Figure 5).

The drug eluting from the 133.2 µg/ml and 2mg/ml loaded microspheres was still below the detection limit at 3 hours. For the higher drug loadings, a burst effect is evident in the first few minutes, followed by a prolonged period of slower release. It is surmised that the burst represents the free drug eluting from the water held within the microspheres, whereas the prolonged elution results from the drug that is "bound" into the spheres essentially by ionic interaction between the charged groups. For the highest loading of drug (from the 20mg/ml loading solution), the burst effect represents some 45% of the total drug loading of the spheres, the remainder taking several days to completely elute from the carrier. Studies have shown that 100% of the drug is eventually eluted from the microspheres.

### Example 7: Visualisation of Doxorubicin Sequestration by High AMPS microspheres

To a vial containing ca 0.5g of High AMPS microspheres in the size range 850-1000 µm (hand sieved), 1ml of doxorubicin in phosphate buffered saline PBS (66.6ug/ml) and 3 ml of PBS was added. The microspheres were placed under a CCD camera, and images taken every 2 mins for a period of 2.5hrs. No agitation of the sample occurred in this time period, but small movements were observed due to localised thermal heating from the light source. The initial and final microspheres are thus identical, and can be compared over the time period. The uptake of drug was observed by the increase in red colour in the microspheres, and the depletion of the surrounding solution (Figure 6):

### Example 8: Preparation of Dried Drug-Loaded Microspheres

Microspheres can be loaded with doxorubicin by the method outlined in example 2. The microspheres are dehydrated using the following procedure: The microspheres to be dehydrated were placed in a plastic container and covered with a 10% acetone (ROMIL) solution made in PBS (Inverclyde Biologicals). The microspheres were left in the solution for 10 minutes during which time they were agitated for 30 seconds several times. The solution was then decanted off and the process repeated twice more. This procedure was repeated with increasing acetone concentrations of 25%, 50%, 75% and finally 100%. After the final 100% dehydration step the acetone was decanted off and the beads placed in an oven set to 50°C and dried to constant mass.

The resulting dried product can be resuspended /rehydrated in saline/contrast media prior to the embolisation procedure. Hydration is rapid taking only a few minutes to swell to >80% of the fully hydrated size.

### Example 9 - Preparation of microsphere slurry

High AMPS microspheres produced according to Example 1 above, are swollen in a solution of 20 mg/ml doxorubicin in water for a period of 30 minutes. The extra-particulate liquid was seen to be substantially decoloured after this period, the colour (red) being substantially localised within the microspheres. The suspension was filtered through a sinterglass funnel to remove supernatant. The microspheres were washed with double their volume of distilled water while on the filter, under slight negative pressure. The microspheres were then transferred into a jar and pumped from the jar into a glass syringe using a peristaltic pump. Following removal of the pump the syringe was closed with a syringe lock and sterilised by gamma irradiation.

### Example 10: Loading - Target vs Actual Loaded Dose

A series of doxorubicin solutions were prepared from 22-80mg/ml in water. 1 ml of these solutions were added to 1 ml of high AMPS microspheres, and uptake monitored by UV. The samples were agitated on a roller mixer. Time points were taken at 10, 20, 30, 60, mins and then at 2hr, out to 24hr. Uptake was calculated from the doxorubicin remaining in solution. The microspheres could be loaded with different doses up to 80mg per ml of hydrated microspheres, and in less than 30 minutes, 99 % of the drug solution is located in the microspheres.

### Example 11: High Dose Doxorubicin

In Example 10 a 80mg/ml solution of doxorubicin was prepared. This was a thick gelatinous mixture, that would not be suitable for everyday use. This high dose was repeated, but using 4 ml of a 20mg/ml Doxorubicin solution. Uptake was monitored by UV, and a final loading of 80mg of drug into 1 ml of hydrated high AMPS microspheres was again achieved.

### Example 12: Loading - Drug Sources

Three sources of doxorubicin were used to prepare microspheres of the present invention with a loading of 25mg/ml.
- Adriamycin^{™} PFS is a commercially (Pharmacia and Upjohn) available solution at a concentration of 2mg/ml.
- Adriamycin^{™} RDF is a commercially (Pharmacia and Upjohn) powder formulation with lactose added for dissolution ease.
- Doxorubicin EP.

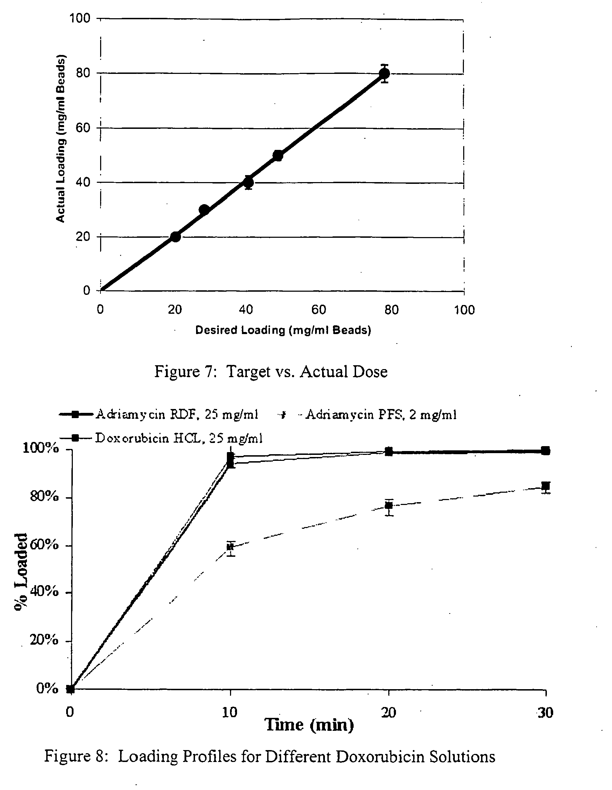

For the Adriamycin RDF and Doxorubicin EP solution, 2ml was added to 2 ml of microspheres, and the uptake monitored by UV. For the Adriamycin PFS solution 50ml of the 2mg/ml solution was added to 2ml of microspheres, and uptake monitored. After 30min both 25mg/ml solutions were fully loaded, the 2mg/ml solution was followed for 24hr to show full uptake (fig 8).

### Example 13: Loading of Other Anthracyclines

Four samples of 1 ml of hydrated high AMPS microspheres (900-1200µm) into an 8ml- glass container were prepared. 1 ml of microspheres in Phosphate buffered saline (PBS), measured with a 10ml- glass cylinder, was transferred to a glass container. Following this, all the PBS was removed with a Pasteur glass pipette in each sample. Loading solutions were prepared by the following: 1 vial of 20 mg of Daunorubicin (Beacon Pharmaceuticals) was reconstituted with 1 ml of water (ROMIL) to give a final concentration of 20mg/ml. 1 vial of 50 mg of epirubicin rapid dissolution (Pharmacia) was reconstituted with 2.5 ml of water to give a final concentration of 20 mg/ml. A solution of 20-mg/ml doxorubicin (Dabur Oncology) was prepared for comparison as in the previous examples. Once prepared, the absorbances of the solutions were read by UV at 483 nm and dilutions were made to produce a standard curve for each drug solution.

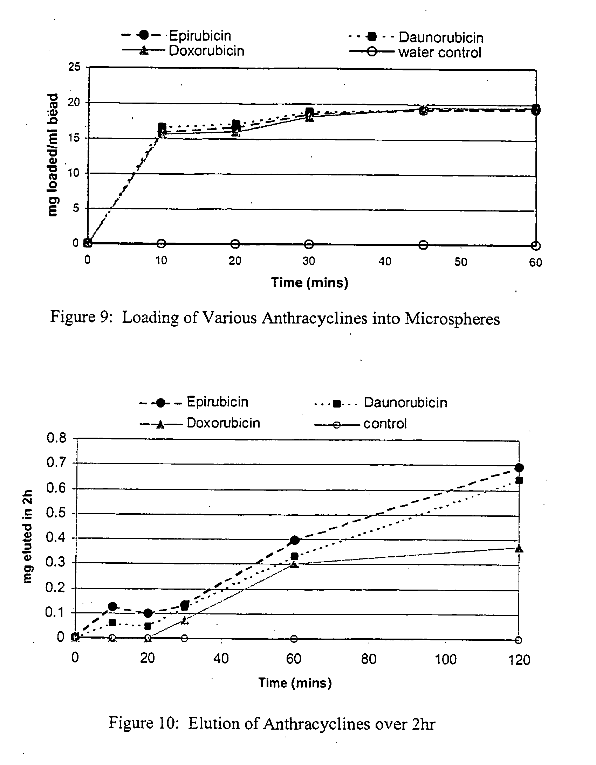

1ml of each loading solution and 1 ml of water (as control) was added to each vial containing 1 ml of microspheres as prepared above and timing was started. The vials were placed on the roller mixer for the entire experiment. At predetermined time points (0, 10, 20, 30, 45 and 60 min) 50 µl was removed, diluted as necessary and read at 483 nm. From these readings, the concentration of the solution at each time point was calculated from the corresponding standard curve in each case. The amount of drug loaded into the microspheres was measured by the depletion of the drug in solution when extracted with the device. From the data the mg drug loaded per 1 ml of hydrated microspheres was calculated and the graph plotted (fig 9). This shows the anthracyclines tested loaded in the same manner.

### Example 14: Elution of Other Anthracyclines

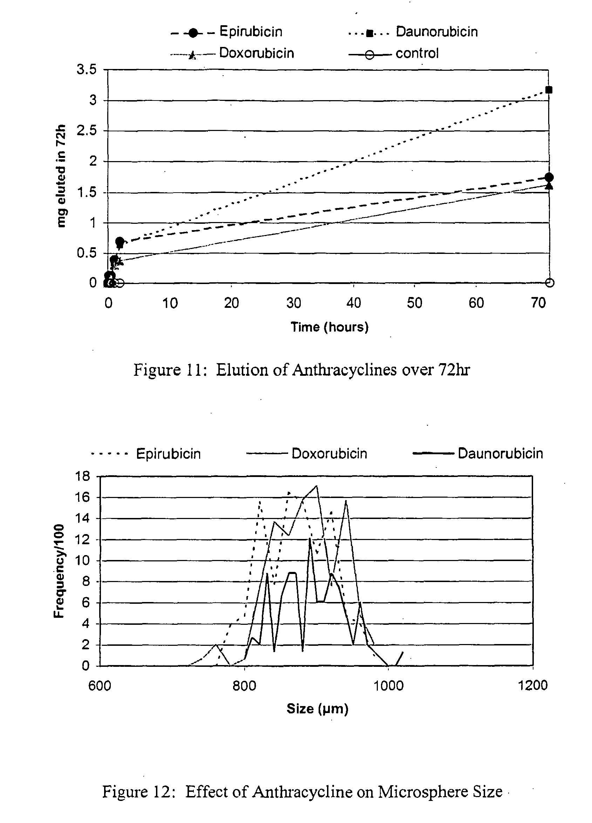

Microspheres loaded as described in example 13 were used to determine drug release profiles. 1 ml of each drug loaded microsphere type was transferred into a brown glass container filled with 100 ml of PBS and timing was started. The containers were placed in a water bath at 37 °C for the entire experiment. At predetermined time points (0, 0.16, 0.5, 1, 2 and 72 hours) 1 ml of the solution was removed, read and then placed back into the container, so the volume remained constant. Samples were read at 483 nm and concentrations were calculated from the equation of the respective anthracycline standard curve determined in example 12. From the data the mg of drug eluted per 1 ml of microspheres were calculated and the graph plotted (fig 10+11). This showed the anthracyclines under study eluted from the microspheres with the same elution profile.

### Example 15: Effect of Anthracyclines on Microsphere Size

Microspheres as described in example 13 were used and size distributions were determined using images of microspheres photographed using a CCD camera and microscope then the diameters resolved with Image Pro Plus 4.05. Microspheres loaded with different anthracyclines were transferred to small cell culture flasks; between 50 and 1500 microspheres were photographed per image. Image Pro Plus 4.05 resolved the diameters of between 100 and 1500 microspheres dependent on size range. Diameters were tabulated and converted to histograms of size range versus frequency, normalised and represented as graphs using Excel (fig 12). This showed the anthracyclines had the same effect on microsphere size.

### Example 16: Drug Loading of Other Commercial Microspheres

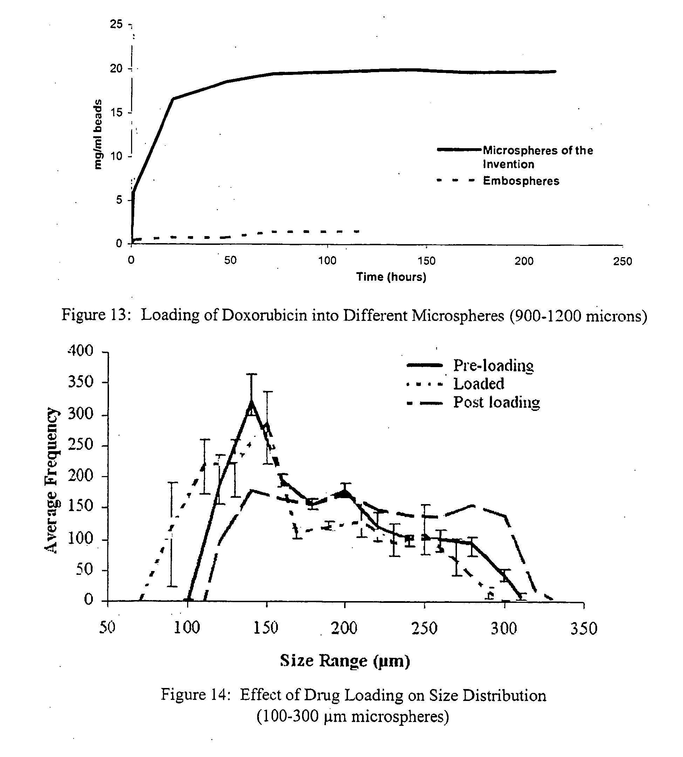

A comparison was made of doxorubicin loading into microspheres of the present invention compared to another commercially-available embolic microsphere (Embosphere, trisacryl microsphere coated with collagen), following the procedures outlines in the previous examples. Clearly, from the figure 13, the microspheres of the present invention demonstrate the capability to sequester the drug, whereas the standard commercial product does not.

### Example 17: Loading - Physical Effects

The effect of loading and elution of doxorubicin on high AMPS microspheres was evaluated by measurement of size and compression after loading and elution of the drug, and deliverability of the doxorubicin loaded microspheres. The loading of drug produced a small decrease in the overall size range, as drug is effectively displacing water from the hydrated spheres (fig 14); this is accompanied by a small decrease in the compressibility. Upon elution it was seen that the size was not permanently affected and nor was compressibility (as determined by Young's Modulus measurement using an Instron tensile tester. The deliverability of the spheres through standard catheters remained unchanged by the process of drug loading.

### Example 18: Elution - Effect of Bead Size

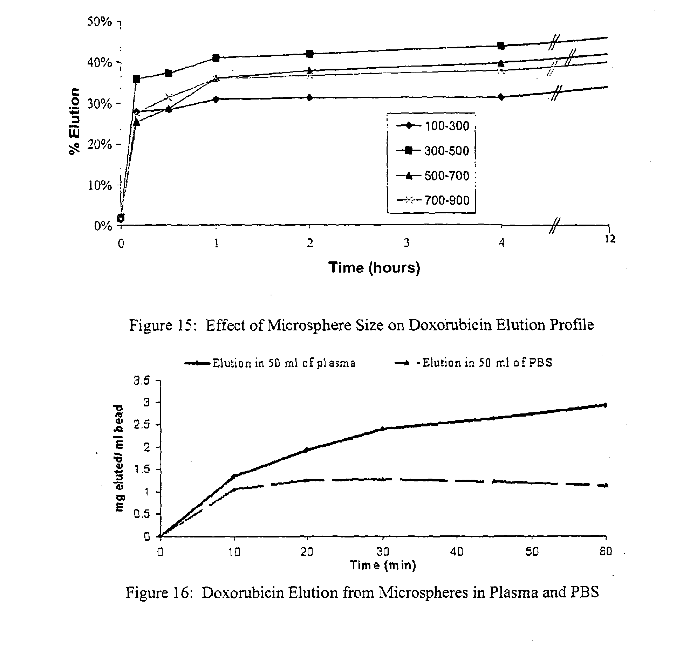

Microspheres of the present invention of each size were loaded with 70mg/ml of doxorubicin solution. Microspheres were then placed in 500ml of phosphate buffered saline, and release measured by UV. The in vitro elution profiles show that up to 40% of the loaded doxorubicin is released in the first 2 hours of elution as a burst, then the remaining drug elutes over at least a 12-day period. All size ranges release with similar characteristics and within ± 5% (fig 15).

### Example 19: Elution - Effect of Media

Microspheres of the present invention loaded with 25mg/ml of doxorubicin were placed in various media and the elution monitored over 60 minutes. Plasma and PBS show slow release over the first 60 minutes. Release into water was below the detection limits of the UV. This suggests that the release of the drug is determinant on the presence of ions to displace the ionically-bound drug from the anionically-charged polymer matrix (fig 16).

### Example 20: Doxorubicin Stability

The effect of the loading and release process on the stability of the drug was determined. The stability of Doxorubicin solution when stored at different conditions was determined. The loading and release of doxorubicin from the microspheres of the present invention were followed by HPLC using the USP method, to determine if the doxorubicin was affected by the process. The resulting chromatograms all show a single peak with similar retention times, showing that there is no detrimental effect on doxorubicin during loading and release from the microspheres.

### Example 21: Doxorubicin-loaded Microspheres - Materials Compatibility

Microspheres of the present invention were loaded with doxorubicin at 25mg/ml. They were then suspended in contrast media and saline, and then left in a deliver catheter (Progreat^{™}, Terumo) and syringe (Merit) for 24 hrs. AT various time points, the stability of doxorubicin, and components were measured (UV/HPLC for Doxorubicin, and Visual inspection/SEM for components). No degradation was observed in components or drug over 24 hours at room temperature.

### Example 22: Preloaded Product - Loaded Dose

Samples were prepared at set doses 5, 10, 20, 45 mg/ml across the size range of microspheres of the present invention. Loading for each sample was determined by UV measurements. The data for 25 separate runs are presented in the following table:

**Table 1: Actual Dose from UV measurement of the Loading Solution.**

| TARGET DOSE mg/ml | 45.0 | 45.0 | 45.0 | 45.0 | 45.0 | 45.0 | 45.0 | 45.0 | 45.0 | |
|---|---|---|---|---|---|---|---|---|---|---|
| Mean Dose mg/ml | 44.79 | 45.25 | 46.60 | 46.56 | 45.66 | 46.56 | 45.74 | 46.57 | 44.025 | |
| SD | 0.42 | 0.45 | 0.02 | 0.06 | 0.06 | 0.02 | 0.02 | 0.02 | 0.0397 | |
| %CV | 0.93 | 1.00 | 0.05 | 0.12 | 0.14 | 0.04 | 0.04 | 0.04 | 0.0901 | |
| Microsphere Size µm | 100-300 | 100- 300 | 300- 500 | 500- 700 | 500- 700 | 700-900 | 700- 900 | 700- 900 | 900-1200 | |
| N° Measurements | 30 | 28 | 9 | 15 | 23 | 24 | 13 | 15 | 5 | |
| | | | | | | | | | | |
| TARGET DOSE mg/ml | 20.0 | 20.0 | 20.0 | 20.0 | 20.0 | 20.0 | | | | |
| Mean Dose mg/ml | 21.31 | 21.12 | 21.05 | 21.04 | 21.02 | 19.65 | | | | |
| SD | 0.01 | 0.01 | 0.03 | 0.02 | 0.03 | 0.0099 | | | | |
| %CV | 0.04 | 0.05 | 0.15 | 0.09 | 0.16 | 0.0504 | | | | |
| Microsphere Size µm | 100-300 | 100- 300 | 300- 500 | 500- 700 | 700- 900 | 900-1200 | | | | |
| N° Measurements | 33 | 28 | 9 | 9 | 9 | 5 | | | | |
| | | | | | | | | | | |
| TARGET DOSE mg/ml | 5.0 | 5.0 | 5.0 | 5.0 | 5.0 | 10.0 | 10.0 | 10.0 | 10.0 | 10.0 |
| Mean Dose mg/ml | 4.99 | 5.14 | 4.98 | 4.98 | 5.13 | 9.98 | 9.98 | 9.96 | 9.93 | 9.93 |
| SD | 0.01 | 0.006 | 0.00 | 0.01 | 0.009 | 0.00 | 0.00 | 0.01 | 0.009 | 0.007 |
| %CV | 0.12 | 0.12 | 0.08 | 0.21 | 0.18 | 0.01 | 0.04 | 0.11 | 0.09 | 0.07 |
| Microsphere Size µm | 100-300 | 300- 500 | 500- 700 | 700- 900 | 900- 1200 | 100-300 | 300- 500 | 500- 700 | 700-900 | 900-1200 |
| N° Measurements | 5 | 5 | 5 | 5 | 5 | 5 | 5 | 5 | 5 | 5 |

The measured dose ranges are:

| | |
|---|---|
| 45mg/ml : | 44.37- 45.77mg/ml (3.11 % Range). |
| 20mg/ml : | 21.11- 21.32 mg/ml (1.05 % Range) |
| 10mg/ml : | 9.93 - 9.98 mg/ml (0.5% Range) |
| 5mg/ml : | 4.98 - 5.14 mg/ml (3.2% Range) |

These data demonstrate that there is little variation between runs and that precise and accurate drug loadings can be achieved.

### Example 23: Preloaded Product - Lyophilisation Weight Loss

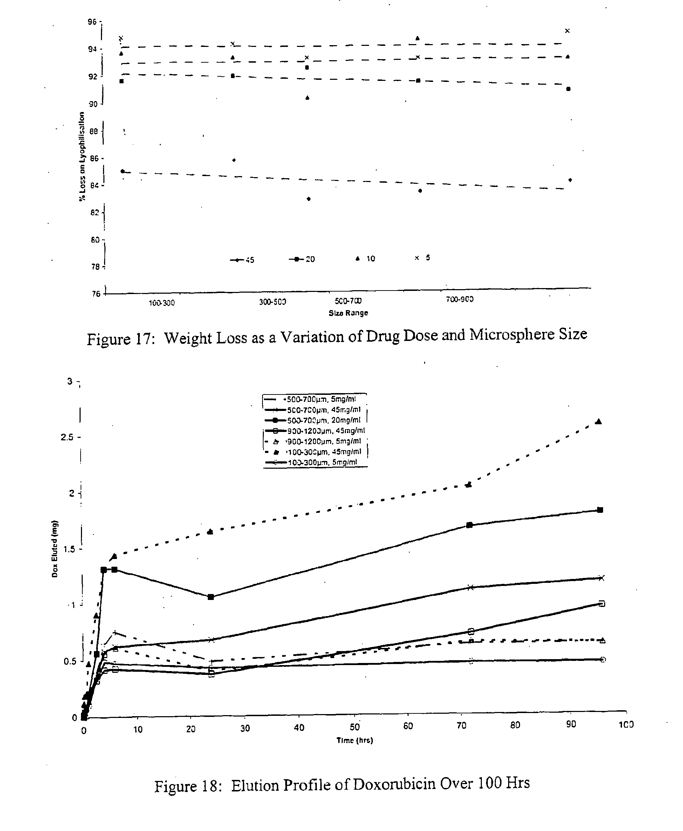

Doxorubicin loaded microspheres of the present invention were subjected to lyophilisation using a proprietary cycle: Percent weight loss was determined for doses of 5, 10, 20 and 45mg/ml for all of the microsphere size ranges (expressed as a % of the loaded microspheres, table 2) for 25 separate runs. A consistent weight loss was obtained, indicating that any variation in the weight of loaded microspheres prior to lyophilisation had no effect on the product post lyophilisation. The data in figure17 show that there is consistently greater than 82% weight reduction on lyophilisation due to water loss.

**Table 2: Weight Loss on Lyophilisation for Doxorubicin Loaded Microspheres**

| **DOSE mg/ml** | **45** | **45** | **45** | **45** | **45** | **45** | **45** | **45** | **45** | |
|---|---|---|---|---|---|---|---|---|---|---|
| Microsphere Size µm | 100-300 | 100-300 | 300-500 | 500-700 | 500-700 | 700-900 | 700-900 | 700- 900 | 900-1200 | |
| Mean loss % | 85.7 | 84.4 | 85.76 | 84.10 | 81.57 | 84.51 | 82.1 | 86.82 | 83.98 | |
| SD | 1.4 | 3.5 | 3.89 | 1.69 | 1.53 | 3.88 | 1.9 | 1.90 | 3.99 | |
| %CV | 1.7 | 4.2 | 4.53 | 2.01 | 1.87 | 4.59 | 2.3 | 2.18 | 4.75 | |
| N° Measurements | 30 | 28 | 9 | 15 | 23 | 24 | 13 | 15 | 5 | |
| | | | | | | | | | | |
| DOSE mg/ml | 20.0 | 20.0 | 20.0 | 20.0 | 20.0 | 20.0 | | | | |
| Microsphere Size µm | 100-300 | 100- 300 | 300- 500 | 500- 700 | 700- 900 | 900-1200 | | | | |
| Mean loss % | 90.6 | 92.7 | 91.92 | 92.48 | 91.38 | 90.65 | | | | |
| SD | 3.3 | 1.3 | 5.03 | 1.35 | 2.34 | 0.85 | | | | |
| %CV | 3.6 | 1.4 | 5.48 | 1.46 | 2.56 | 0.94 | | | | |
| N° Measurements | 33 | 28 | 9 | 9 | 9 | 5 | | | | |
| | | | | | | | | | | |
| DOSE mg/ml | 5.0 | 5.0 | 5.0 | 5.0 | 5.0 | 10.0 | 10.0 | 10.0 | 10.0 | 10.0 |
| Microsphere Size µm | 100-300 | 300- 500 | 500- 700 | 700- 900 | 900- 1200 | 100- 300 | 300- 500 | 500- 700 | 700- 900 | 900-1200 |
| Mean loss % | 94.8 | 94.26 | 93.2 | 93.1 | 94.90 | 93.7 | 93.30 | 90.3 | 94.5 | 93.04 |
| SD | 0.2 | 1.11 | 0.5 | 0.5 | 0.78 | 0.2 | 0.26 | 8.3 | 0.2 | 0.29 |
| %CV | 0.2 | 1.18 | 0.6 | 0.6 | 0.82 | 0.2 | 0.28 | 9.2 | 0.2 | 0.31 |
| N° Measurements | 5 | 5 | 5 | 5 | 5 | 5 | 5 | 5 | 5 | 5 |

### Example 24: Preloaded Product - Residual Water Content

Microspheres of the present invention loaded with doxorubicin at 5, 10, 20 and 45 mg/ml across the entire size range were prepared, lyophilised and then subjected to gamma irradiation for sterilisation. The residual water content of the samples was then determined by a gravimetric method involving heating the microspheres at 70°C until a constant weight was achieved. A residual water content of less than 5% was determined for all samples.

**Table 3**

| | *Drug Dose mg*/*ml* | | | | |
|---|---|---|---|---|---|
| Drug Dose mg/ml | 0 | 5 | 10 | 20 | 45 |

| Size Range | Residual Water % | | | | |
|---|---|---|---|---|---|
| 100-300 µm | 2.65 | 2.10 | 2.46 | 3.70 | 3.52 |
| 300-500 µm | 2.74 | 2.13 | 2.17 | 1.60 | 1.86 |
| 500-700 µm - | 2.68 | 2.34 | 2.58 | 0.90 | 4.50 |
| 700-900 µm | 2.83 | 2.42 | 2.51 | 1.07 | 1.40 |
| 900-1200 µm | 2.71 | 1.64 | 2.71 | 2.88 | 2.85 |

### Example 25: Preloaded Product- Release Following Rehydration

Microspheres of the present invention loaded with doxorubicin at 5, 20 and 45 mg/ml across the entire size range were prepared, lyophilised and then subjected to gamma irradiation. Samples were then re-hydrated in water, and the release of the drug into PBS followed by UV out to 100hr (fig 18).

### Example 26: Size of Rehydrated Doxorubicin-loaded Microspheres

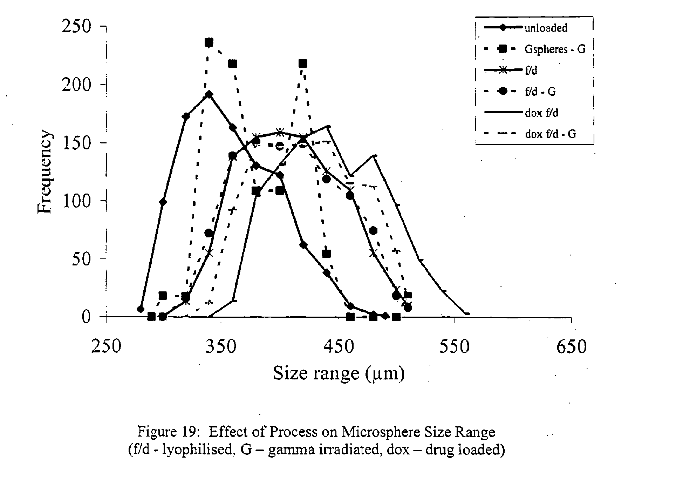

Microspheres of the present invention (300-500µm) loaded with doxorubicin at 20mg/ml were prepared, lyophilised and some samples then subjected to gamma irradiation. Samples were re-hydrated in water, and then sizing carried out using calibrated image analysis equipment (Image Pro-Plus, fig 19).

Non-drug loaded samples were likewise treated and sized. The data in figure 19 show there are some slight shifts in size upon various treatments, but non that would take the product outside of the acceptable specification range of 250-500µm.

### Example 27: Sustained Release of Doxorubicin From Doxorubicin-loaded Microspheres Following TranscatheterArterial Chemoembolisation in a Rabbit Model of Liver Cancer (Vx-2)

The objective of this study was to evaluate the performance of Doxorubicin-loaded microspheres of the present invention in a rabbit liver cancer model delivered via Transcatheter Arterial Chemoembolisation (TACE). The study was carried out at The John Hopkins Hospital, Baltimore.

### 27.1 Materials and Methods:

Animals were divided into 6 groups (groups 1,2,3,4,5,6) of 5 animals each (4 study animals, 1 control). Control animals in all groups received an intraarterial injection of doxorubicin (same concentration as the treated animals), whereas treatment animals were treated with modified chemoembolisation protocol with the drug eluting spheres containing doxorubicin. Animals in the various groups were sacrificed at the following time points:
Group 1: 1 hour after the chemoembolisation procedure
Group 2: 12 hours after the chemoembolisation procedure
Group 3: 24 hours after the chemoembolisation procedure
Group 4: 3 days after the chemoembolisation procedure
Group 5: 7 days after the chemoembolisation procedure
Group 6: 14 days after the chemoembolisation procedure

### 27.2 Animal Preparation:

The VX2 tumour cell line were injected into the hindlegs of carrier rabbits (New Zealand White) and grown for 14 days. Resultant tumours were harvested from each rabbit carrier and a tumour brei prepared from each by dissection of viable tumour tissue, aseptic mincing, and passage through a stainless steel sieve. The rabbits were pre-anesthetized with a mixture of intramuscularacepromazine (1 mg/kg) and Ketamine hydrochloride (20 mg/kg). After approximately 15 minutes, IV access was established via a marginal ear vein and the animal was given sodium Pentothal IV (40mg/kg) to maintain a surgical plan of anesthesia. The abdomen was shaved and prepped with Benzidine and a midline incision made. The liver of each rabbit was exposed by median laporotomy, then an aliquot of brei (0.2 ml) was injected directly using a 21 G angiocath into the left lobe of the exposed liver in order to develop a solitary lesion with adequate surrounding liver parenchyma. One tumour brei was used for each two test rabbits. The tumour was allowed to grow in the rabbit livers for 14 days, to a size based on previous experiments expected to range between 2.5 and 3.5 cm in diameter. Any bleeding was controlled with electrocautery. The abdomen was then closed with running suture, and the skin closed with sutures and bandaged. Proper aseptic techniques were observed throughout the procedure. Following surgery, the animals were placed in cages, kept warm with blankets and monitored for end tidal CO2 until they recover from anesthesia. Analgesic buprenorphine 0.02-0.05 mg/kg administered SQ q12 hours for 3 days was given if it was apparent that the animals were in pain or physical distress.

### 27.3 Preparation of Doxorubicin Eluting Microspheres:

Microspheres of the present invention of the size range 100-300 microns and loaded with 45mg/ml of doxorubicin were prepared as in example 22, lyophilised as in example 23 and sterilised using gamma irradiation. Immediately prior to use, the microspheres were hydrated in 1 ml of sterile water, to which 2 ml of omnipaque and 1ml of saline were added. The solution was prepared at least 10 minutes before it was to be injected and 1 ml of the total solution was injected intraarterially in each rabbit (as described below).

### 27.4 Chemoembolisation Procedure:

Two weeks after implantation of the tumour in the rabbit liver, the animals were brought back for "chemoembolisation". Administration of preanesthesia, IV access and sodium Pentothal anesthesia were carried out as described above. Access was obtained into the common femoral artery, after which a catheter was manipulated into the common hepatic artery. Injection of contrast demonstrated the location of the tumour after which a 2F JB1 catheter was advanced as close to the tumour as possible. If necessary, a Transsend guide wire was used to guide the catheter into the target artery. Once the catheter was adequately positioned, the doxorubicin eluting spheres were injected into the tumour bed as describes above. Controls were injected with equivalent concentration of doxorubicin only without embolisation. After completion of the "chemoembolisation", the catheter was removed, and the artery ligated using resorbable suture material to obtain hemostasis. Proper aseptic techniques were observed throughout the procedure and post procedure. Every effort was made to minimize discomfort and pain including limiting the surgical incision for tumour implantation, subcutaneous injections of buprenorphine for minimizing pain, placing clinical calls whenever required for evaluation of the animal's condition and level of pain and discomfort. The animals were then returned to their cages. All animals were sacrificed according to the time points described above.

Animals were sacrificed according to veterinary regulatory rules. All animals in each animal group as per its time point after treatment were sacrificed under deep anesthesia by IV injections of 100mg/kg of thiopental IV.

### 27.5 Pathological and Histological Evaluation:

The liver of the rabbits were dissected, carefully removed and placed in a container containing 5% formaldehyde. Livers were sliced at 5mm intervals for gross examination. Each section was embedded completely in paraffin, after which 4 µm sliced and treated with H&E stain. Tumour viability was estimated by visual inspection and expressed as a percentage of viable tumour area for each slice. Concentration of doxorubicin within the tumour and nontumourous liver tissue was determined using HPLC.

### 27.6 Blood Collection for Doxorubicin Analysis:

3ml of whole blood was collected at 20', 40', 60', 120' and 180' each, from the time of injection of the drug eluting beads, via an arterial catheter inserted in ear. It was then transferred to a heparinized vacutainer and centrifuged at 2000g for 10 minutes at room temperature. Plasma was separated out and transferred to a labeled polypropylene capped tube. It was the snap frozen in methanol/ice. And stored at -20°C until time of analytical analysis.

### 27.7 Processing of Tissue for Determination of Doxorubicin Concentrations in Tumour and Non-tumourous Liver Tissue:

Tumour and non-tumourous liver tissue (approx 100mg) were excised and removed from the overlying skin and debris. The weight of the tissue was accurately determined and recorded using a preweighted tube and immediately placed on dry ice, and later stored at -80°C until time of analytical analysis.

### 27.8 Results:

### Drug Distribution Summary:

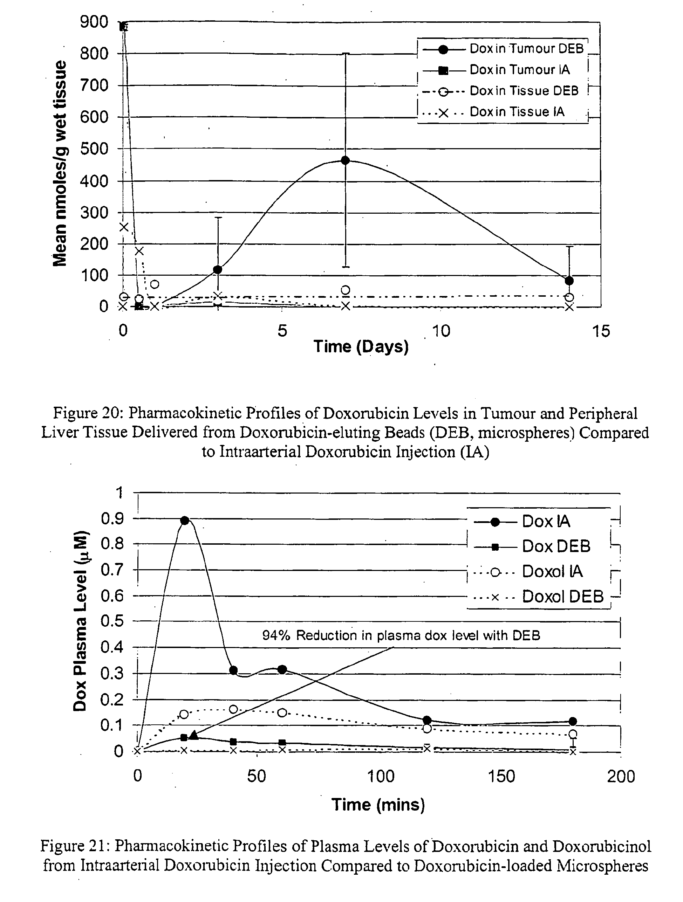

1. The greatest concentration of doxorubicin within the tumour was obtained 7 days post treatment, followed by the 3-day group (Figure 20).
2. Concentration of doxorubicin within the tumour remained high even in the 14-day group, suggesting continuous elution of doxorubicin from the beads (Figure 20).
3. Minimal concentration of doxorubicin and breakdown products (Doxorubicinol), within plasma at all time points. This is especially striking 20 minutes after administration and the doxorubicin concentration in plasma continues to decline almost linearly from 20 to 180 minutes. Doxorubicin concentration was between 10 and 17 times greater in plasma when injected intraarterially without the beads (Figure 21).

### Histological Observations:

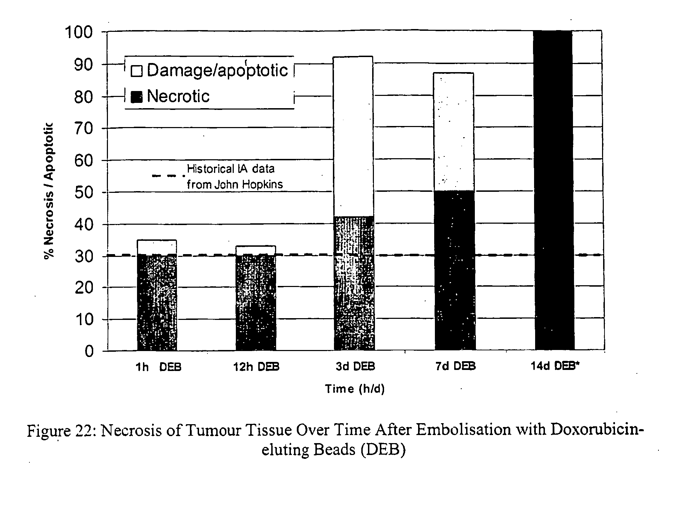

1. Tumor necrosis was greatest at 7-14 days (Figure 22).
2. Note that the 1- and 12 hour groups can be considered control since it is too soon for the chemotherapeutic agents to start killing cells.
3. Finally, 50% and 37% of the cancer cells were neither completely dead nor completely viable in the 3- and 7 day groups respectively. Those cells are likely "damaged" or perhaps even apoptotic.
4. Regarding the distribution of the beads, none were found in the control animals, as expected. In the study animals, the beads were all found to have remained within the arterioles at the expected vessel size i.e 100 to 300 microns. No beads escaped the intravascular space.
5. It appears that in the 14-day group most of the liver tissue on the left side, where the tumor had been implanted, was necrotic. Distinction between dead cancer and normal cells was very difficult to establish. Hence, we can assume that the potency of the drug-eluting beads was at its maximum after 14 days. These results were consistent.
6. Histologic analysis also demonstrated the efficacy of the drug-eluting beads in killing cancer cells. We know from multiple previous experiments that intraarterial injections of doxorubicin or carboplatin (in the same concentrations) only result in 30% necrosis of the tumour, which is roughly equal to that of untreated (i.e control) animals. In contrast, treatment of the rabbits with the drug-eluting beads caused between 50 and 100% necrosis, with necrosis being most complete at 14 days, which is significantly greater than the intraarterial injection alone (Figure 22).

### Example 28 - Clinical trials

Slurries of microspheres made according to Example 9 are used in a clinical trial to assess the safety, pharmaco kinetic profile and efficacy in the treatment of patients with unresectable hepatocellular carcinoma (HCC). The patients eligible for the study 18 to 75 years of age, with HCC not suitable for radical therapies such as resection, liver transplantation, percutaneous therapies, with well-preserved liver function (Child-Pugh A class), without any liver decompensation. Excluded are:
a) Patients previously treated with any anti-cancer therapy for HCC;
b) Patients with another primary tumour;
c) Patients with advanced liver disease: Child-Pugh's B-C class or active gastrointestinal bleeding, encephalopathy or ascites. Bilirubin levels > 3 mg/dL;
d) Patients with tumoral disease: BCLC class C (vascular invasion -including segmental portal obstruction-, extrahepatic spread or cancer-related symptoms=PST of 1-4) or D class (WHO performance status 3 or 4, Okuda III stage);
e) Patients having any contraindication for hepatic embolization procedures: porto-systemic shunt, hepatofugal blood flow; impaired clotting tests (platelet count < 50.000/mm³ or prothrombin activity < 50 percent), renal failure, severe atheromatosis; and
f) Patients with any contraindication for doxorubicin administration (serum bilirubin ≥ 5 mg/dL, leukocyte count ≤ 3.000 cells/mm³, cardiac ejection fraction < 50 percent).

Chemoembolization will be performed at time 0 and month 2. Treatment will be discontinued upon development of any exclusion criteria or as a result of the patients' decision. Embolization will be performed by injecting doxorubicin-loaded microspheres admixed with contrast agent immediately before administration until achieving flow stagnation. Diameter of the PVA microspheres will be selected by the surgeon and will probably have sizes around 500 µm. No antibiotic prophylaxis will be used.

The escalation dose to analyse the pharmacokinetic profile of doxorubicin-microspheres will start for bilirubin levels < 1.5 mg/dL with 25 mg/m² per treatment (2 patients), 50 mg/m² (2 patients), 75 mg/m² (2 patients) 100 mg/m² (2 patients). For patients with bilirubin levels of 1.5-3 mg/dL it will start at 25 mg/m² (2 patients), 50 mg/m² (2 patients), 75 mg/m² (2 patients) 100 mg/m² (2 patients). If no does-limiting toxicity is seen at a dose level, the dose is escalated for the next group, with a maximum total dose of 150 mg doxorubicin in a single treatment.

Pharmacokinetic assessment: Samples for doxorubicin levels will be taken from peripheral blood at 1 h, 6h, 24h, 48h and 7 days after the procedure, either during hospital stay or in the outpatient clinic.

Safety: Treatment-related complications will be recorded and analysed throughout the 6-month study period. Adverse events will be recorded during the hospital stay (4 days) at month 0 and 2. The patients will be visited in the outpatient clinic at day 7th, day 14th and month 1, 3 and 6, by means of clinical examination and laboratory parameters. Adverse events will be elicited at all visits following start of treatment. This includes registration of myelosupression and other doxorubicin-related toxicities, liver failure, renal failure, infections - cholecystitis, liver abscess, spontaneous bacterial peritonitis, bacteremia, ischemic hepatitis or biliary stricture, gastrointestinal haemorrhage.

Efficacy: Objective responses will be assessed by contrast-enhanced spiral computed tomography at month three and six. Its magnitude is defined according to the EASL Consensus criteria: Complete response: no evidence of neoplastic disease; Partial response: reduction in total tumour load of >50 percent; No change: reduction of <50 percent or increase of <25 percent; Progressive disease: increase of >25 percent. Thus, objective responses accounted for complete and partial responses.

## Claims

1. A composition comprising particles having a matrix of water-swellable water-insoluble polymer and, absorbed in the matrix, a water-soluble therapeutic agent, and is **characterised in that** the polymer has an anionic charge in the range 0.1 to 10 meq g⁻¹ at a pH in the range 6 to 8 and comprises covalently cross-linked poly(vinyl alcohol), **in that** the particles, when swollen to equilibrium in water, have particle sizes in the range 40-1500 µm and **in that** the therapeutic agent is an anthracycline compound having at least one amine group.

2. A composition according to claim 1 formed by copolymerising poly (vinyl alcohol) macromer having at least two pendant ethylenically unsaturated groups per molecule with ethylenically unsaturated monomers including anionic monomer.

3. A composition according to claim 2 in which the anionic monomer has the general formula I
Y¹ BQ
in which Y¹ is selected from 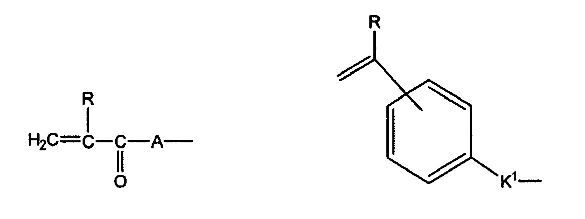 CH₂=C(R)-CH₂-O-, CH₂=C(R)-CH₂ OC(O)-, CH₂=C(R)OC(O)-, CH₂=C(R)-O-, CH₂=C(R)CH₂OC(O)N(R¹)-, R²OOCCR=CRC(O)-O-, RCH=CHC(O)O-, RCH=C(COOR²)CH₂-C(O)-O-, 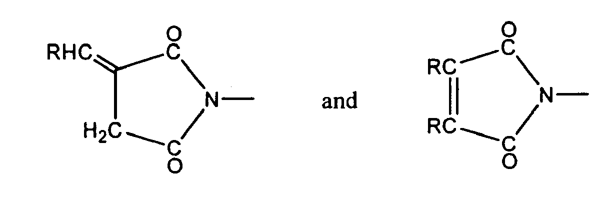 wherein:
R is hydrogen or a C₁-C₄ alkyl group;
R¹ is hydrogen or a C₁-C₄ alkyl group;
R² is hydrogen or a C₁₋₄ alkyl group or BQ where B and Q are as defined below;
A is -O- or -NR¹-;
K¹ is a group -(CH₂)ᵣOC(O)-, -(CH₂)ᵣC(O)O-, - (CH₂)ᵣOC(O)O-, -(CH₂)ᵣNR³-, -(CH₂)ᵣNR³C(O)-, -(CH₂)ᵣC(O)NR³-, -(CH₂)ᵣNR³C(O)O-, -(CH₂)ᵣOC(O)NR³-, -(CH₂)ᵣNR³C(O)NR³- (in which the groups R³ are the same or different), -(CH₂)ᵣO-, -(CH₂)ᵣSO₃ -, or, optionally in combination with B¹, a valence bond and r is from 1 to 12 and R³ is hydrogen or a C₁-C₄ alkyl group;
B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if Q or Y¹ contains a terminal carbon atom bonded to B a valence bond; and
Q is an anionic group.

4. A composition according to claim 3 in which Y¹ is CH₂=CRCOA in which R is H or methyl, A is NH, and in which B is C₁₋₁₂ alkanediyl.

5. A composition according to claim 3 in which Q is a carboxylate, carbonate, sulphonate, sulphate, nitrate, phosphonate or phosphate group, preferably a sulphonate group.

6. A composition according to any preceding claim in which the PVA macromer has average molecular weight in the range 1,000 to 500,000 D preferably in the range 10,000 to 100,000 D.

7. A composition according to any preceding claim in which the pendant ethylenic groups are linked via cyclic acetal linkages with oxygen atoms from adjacent hydroxyl groups, preferably formed by the reaction of N-(alk)acrylamino-substituted aldehyde, usually in the form of the dialkyl acetal, preferably N-acrylaminoacetaldehyde dimethyl acetal.

8. A composition according to any preceding claim in which the polymer has an anionic charge of at least 1.0 meq g⁻¹.

9. A composition according to any preceding claim in which the anthracycline is a compound of the general formula II

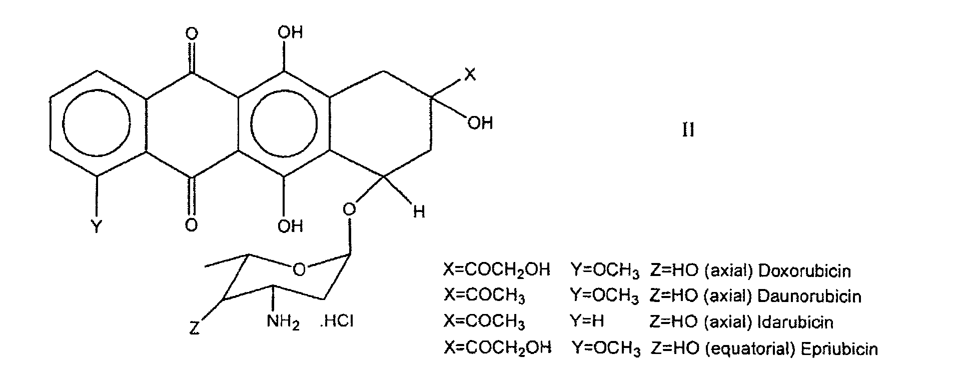

10. A composition according to claim 9 in which the anthracycline is doxorubicin.

11. A composition according to any preceding claim in which the particles are microspheres.

12. A composition according to any preceding claim in which the particles are swollen with and suspended in an aqueous liquid.

13. A composition according to claim 12 further comprising an imaging agent.

14. A composition according to claim 13 in which the imaging agent is a radiopaque agent.

15. A composition according to any of claims 1 to 11 in which the particles are in the form of a pumpable slurry comprising particles swollen with aqueous liquid, substantially free of extra particulate liquid.

16. A composition according to claim 15, which is filled into a storage container, preferably a syringe, and which is sterile.

17. A composition according to any of claims 1 to 11 which is substantially dry.

18. Use of an anthracycline in the manufacture of a composition according to any preceding claim for use in the treatment by embolotherapy of a solid tumour.

19. Process for producing a composition according to any of claims 1 to 17 in which particles of the water-swellable, water-insoluble polymer are contacted with a solution of the anthracycline in the presence of water whereby anthracycline is absorbed into the matrix of the polymer.

20. Process according to claim 19 in which the contacting is carried out by suspending the polymer particles in an aqueous solution of the anthracycline.

21. Process according to claim 20 in which the particles of polymer with anthracycline absorbed in the matrix are recovered from the suspension and dried.

22. Process according to claim 21 in which the particles are dried by freeze-drying.

23. A process according to claim 20 in which the particles of swollen polymer are separated from supernatant liquid and are transferred while still swollen by the swelling liquid into a storage container, preferably a syringe, and sterilised and stored in the container.

24. Use according to claim 18 in which the polymer matrix has been formed by the copolymerisation of a poly(vinyl alcohol) macromer having at least 2 pendant ethylenically unsaturated groups per molecule and an ethylenically unsaturated anionic monomer.

## Patentansprüche

1. Zusammensetzung, umfassend Teilchen mit einer Matrix von in Wasser quellbarem, in Wasser unlöslichem Polymer und, absorbiert in der Matrix, ein wasserlösliches therapeutisches Mittel, **dadurch gekennzeichnet, dass** das Polymer eine anionische Ladung im Bereich von 0,1 bis 10 mÄq. g⁻¹ bei einem pH im Bereich von 6 bis 8 aufweist und kovalent vernetzten Poly(vinylalkohol) umfasst, dass die Teilchen, wenn sie im Gleichgewicht in Wasser gequollen sind, Teilchengrößen im Bereich von 40 bis 1.500 µm aufweisen und dass das therapeutische Mittel eine Anthracyclinverbindung mit mindestens einer Amingruppe ist.

2. Zusammensetzung nach Anspruch 1, gebildet durch Copolymerisieren von Poly(vinylalkohol)-Makromer mit mindestens zwei seitenständigen ethylenisch ungesättigten Gruppen pro Molekül mit ethylenisch ungesättigten Monomeren, einschließlich eines anionischen Monomers.

3. Zusammensetzung nach Anspruch 2, bei der das anionische Monomer die allgemeine Formel I aufweist
Y¹BQ
worin Y¹ ausgewählt ist aus 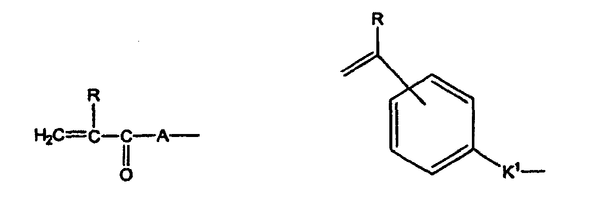 CH₂=C(R)-CH₂-O-, CH₂=C(R)-CH₂ OC(O)-, CH₂=C(R)OC(O)-, CH₂=C(R)-O-, CH₂=C(R)CH₂OC(O)N(R¹)-, R²OOCCR=CRC(O)-O-, RCH=CHC(O)O-, RCH=C(COOR²)CH₂-C(O)-O-, 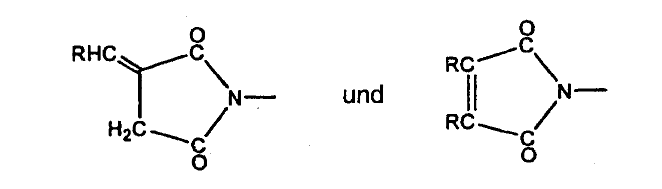 worin:
R Wasserstoff oder eine C₁-C₄-Alkylgruppe ist;
R¹ Wasserstoff oder eine C₁-C₄-Alkylgruppe ist;
R² Wasserstoff oder eine C₁₋₄-Alkylgruppe oder BQ, worin B und Q wie nachstehend definiert sind, ist;
A -O- oder -NR¹- ist;
K¹ eine Gruppe -(CH₂)ᵣOC(O)-, -(CH₂)ᵣC(O)O-, -(CH₂)ᵣOC(O)O-, -(CH₂)ᵣNR³-, -(CH₂)ᵣNR³C(O)-, -(CH₂)ᵣC(O)NR³-, -(CH₂)ᵣNR³C(O)O-, -(CH₂)ᵣOC(O)NR³-, -(CH₂)ᵣNR³C(O)NR³- (worin die Gruppen R³ gleich oder verschieden sind), -(CH₂)ᵣO-, -(CH₂)ᵣSO₃- oder gegebenenfalls in Kombination mit B¹ eine Valenzbindung ist und r 1 bis 12 ist und R³ Wasserstoff oder eine C₁-C₄-Alkylgruppe ist;
B eine geradkettige oder verzweigte Alkandiyl-, Oxaalkylen-, Alkandiyloxaalkandiyl- oder Alkandiyloligo(oxaalkandiyl)-Kette, gegebenenfalls enthaltend ein oder mehrere Fluoratome bis zu und einschließlich perfluorierten Ketten oder, wenn Q oder Y¹ ein an B gebundenes endständiges Kohlenstoffatom enthalten, eine Valenzbindung ist; und
Q eine anionische Gruppe ist.

4. Zusammensetzung nach Anspruch 3, worin Y¹ CH₂=CRCOA ist, worin R H oder Methyl ist, A NH ist und worin B C₁₋₁₂-Alkandiyl ist.

5. Zusammensetzung nach Anspruch 3, worin Q eine Carboxylat-, Carbonat-, Sulfonat-, Sulfat-, Nitrat-, Phosphonat- oder Phosphatgruppe, vorzugsweise eine Sulfonatgruppe, ist.

6. Zusammensetzung nach irgendeinem vorhergehenden Anspruch, bei der das PVA-Makromer ein mittleres Molekulargewicht im Bereich von 1.000 bis 500.000 D, vorzugsweise im Bereich von 10.000 bis 100.000 D, aufweist.

7. Zusammensetzung nach irgendeinem vorhergehenden Anspruch, bei der die seitenständigen ethylenischen Gruppen über cyclische Acetalverknüpfungen mit Sauerstoffatomen von benachbarten Hydroxylgruppen verknüpft sind, vorzugsweise gebildet durch die Reaktion von N-(Alk)acrylamino-substituiertem Aldehyd, gewöhnlich in Form des Dialkylacetals, bevorzugt N-Acrylaminoacetaldehyddimethylacetal.

8. Zusammensetzung nach irgendeinem vorhergehenden Anspruch, bei der das Polymer eine anionische Ladung von mindestens 1,0 mÄq. g⁻¹ aufweist.

9. Zusammensetzung nach irgendeinem vorhergehenden Anspruch, bei der das Anthracyclin eine Verbindung der allgemeinen Formel II ist

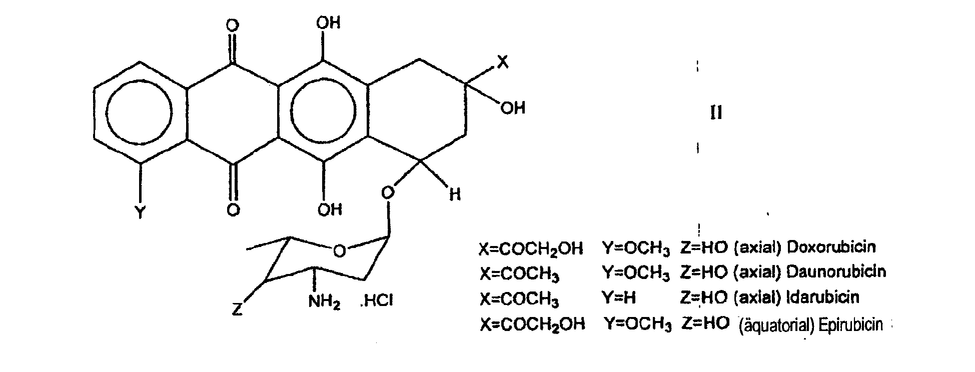

10. Zusammensetzung nach Anspruch 9, bei der das Anthracyclin Doxorubicin ist.

11. Zusammensetzung nach irgendeinem vorhergehenden Anspruch, bei der die Teilchen Mikrokugeln sind.

12. Zusammensetzung nach irgendeinem vorhergehenden Anspruch, bei der die Teilchen mit einer wässrigen Flüssigkeit gequollen und darin suspendiert sind.

13. Zusammensetzung nach Anspruch 12, ferner umfassend ein bildgebendes Mittel.

14. Zusammensetzung nach Anspruch 13, bei der das bildgebende Mittel ein strahlenundurchlässiges Mittel ist.

15. Zusammensetzung nach irgendeinem der Ansprüche 1 bis 11, bei der die Teilchen in Form einer pumpfähigen Aufschlämmung umfassend Teilchen, die mit wässriger Flüssigkeit gequollen sind, im wesentlichen frei von außerpartikulärer Flüssigkeit, vorliegen.

16. Zusammensetzung nach Anspruch 15, die in einen Lagerbehälter, vorzugsweise eine Spritze, gefüllt ist, der steril ist.

17. Zusammensetzung nach irgendeinem der Ansprüche 1 bis 11, die im wesentlichen trocken ist.

18. Verwendung eines Anthracyclins bei der Herstellung einer Zusammensetzung nach irgendeinem vorhergehenden Anspruch zur Verwendung bei der Behandlung durch Embolotherapie eines soliden Tumors.

19. Verfahren zur Herstellung einer Zusammensetzung nach irgendeinem der Ansprüche 1 bis 17, bei dem Teilchen des in Wasser quellbaren, in Wasser unlöslichen Polymers mit einer Lösung des Anthracyclins in Anwesenheit von Wasser kontaktiert werden, wodurch Anthracyclin in die Matrix des Polymers absorbiert wird.

20. Verfahren nach Anspruch 19, bei dem das Kontaktieren durch Suspendieren der Polymerteilchen in einer wässrigen Lösung des Anthracyclins durchgeführt wird.

21. Verfahren nach Anspruch 20, bei dem die Teilchen des Polymers mit in der Matrix absorbiertem Anthracyclin aus der Suspension gewonnen und getrocknet werden.

22. Verfahren nach Anspruch 21, bei dem die Teilchen durch Gefriertrocknen getrocknet werden.

23. Verfahren nach Anspruch 20, bei dem die Teilchen aus gequollenem Polymer von der überstehenden Flüssigkeit getrennt und noch gequollen durch die Quellflüssigkeit in einen Lagerbehälter, bevorzugt eine Spritze, überführt und sterilisiert und in dem Behälter gelagert werden.

24. Verwendung nach Anspruch 18, bei der die Polymermatrix durch die Copolymerisation eines Poly(vinylalkohol)-Makromers mit mindestens 2 seitenständigen ethylenisch ungesättigten Gruppen pro Molekül und einem ethylenisch ungesättigten anionischen Monomer gebildet worden ist.

## Revendications

1. Composition comprenant des particules ayant une matrice de polymère gonflant dans l'eau et insoluble dans l'eau et, absorbé dans la matrice, un agent thérapeutique hydrosoluble, et est **caractérisée en ce que** le polymère a une charge anionique dans la plage de 0,1 à 10 meq g⁻¹ à un pH dans la plage de 6 à 8 et comprend du poly(alcool vinylique) réticulé de manière covalente, **en ce que** les particules, lorsqu'elles sont gonflées à l'équilibre dans l'eau, ont des tailles de particule dans la plage de 40 à 1 500 µm et **en ce que** l'agent thérapeutique est un composé d'anthracycline ayant au moins un groupe amine.

2. Composition selon la revendication 1, formée par copolymérisation d'un macromère de poly(alcool vinylique) ayant au moins deux groupes pendants éthyléniquement insaturés par molécule avec des monomères éthyléniquement insaturés comprenant un monomère anionique.

3. Composition selon la revendication 2, dans laquelle le monomère anionique a la formule générale I
**Y¹BQ**
dans laquelle Y¹ est choisi parmi 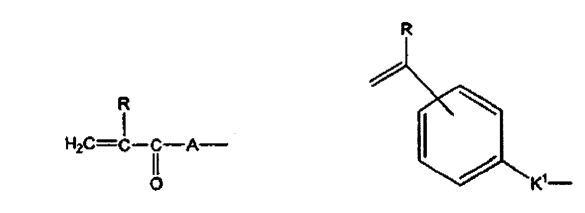 CH₂=C(R)-CH₂-O-,CH₂=C(R)-CH₂OC(O)-, CH₂=C(R)OC(O)-,CH₂=C(R)-O-, CH₂=C(R)CH₂OC(O)N(R¹)-, R²OOCCR=CRC(O)-O-, RCH=CHC(O)O-, RCH=C(COOR²) CH₂-C(O)-O-, 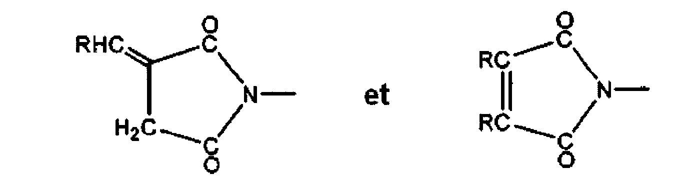 où :
R est un atome d'hydrogène ou un groupe alkyle en C_{1 à 4} ;
R¹ est un atome d'hydrogène ou un groupe alkyle en C_{1 à 4};
R² est un atome d'hydrogène ou un groupe alkyle en C_{1 à 4} ou BQ où B et Q sont définis ci-dessous ;
A est -O- ou -NR¹- ;
K¹ est un groupe (CH₂)ᵣOC(O)-,-(CH₂)ᵣC(O)O-, -(CH₂)ᵣOC(O)O-, -(CH₂)ᵣNR³-, -(CH₂)ᵣNR³C(O)-, -(CH₂)ᵣC(O)NR³-, -(CH₂)ᵣNR³C(O)O-, -(CH₂)ᵣOC(O)NR³-, - (CH₂)ᵣNR³C(O)NR³- (dans lequel les groupes R³ sont identiques ou différents), -(CH₂)ᵣO-, - (CH₂)ᵣSO₃-, ou, facultativement en combinaison avec B¹, une liaison de valence et r vaut de 1 à 12 et R³ est un atome d'hydrogène ou un groupe alkyle en C₁ à C₄ ;
B est une chaîne alcanediyle, oxaalkylène, alcanediyloxaalcanediyle, ou alcanediyloligo(oxaalcanediyle) droite ou ramifiée contenant facultativement un ou plusieurs atomes de fluor jusqu'à des chaînes perfluorées et comprenant celles-ci ou, si Q ou Y¹ contient un atome de carbone terminal lié à B par une liaison de valence ; et
Q est un groupe anionique.

4. Composition selon la revendication 3, dans laquelle Y¹est CH₂=CRCOA où R est H ou méthyle, A est NH et où B est un alcanediyle en C_{1 à 12}.

5. Composition selon la revendication 3, dans laquelle Q est un groupe carboxylate, carbonate, sulfonate, sulfate, nitrate, phosphonate ou un groupe phosphate, de préférence un groupe sulfonate.

6. Composition selon l'une quelconque des revendications précédentes, dans laquelle le macromère de PVA a un poids moléculaire moyen dans la plage de 1 000 à 500 000 D, de préférence dans la plage de 10 000 à 100 000 D.

7. Composition selon l'une quelconque des revendications précédentes, dans laquelle les groupes éthyléniques pendants sont liés via des liaisons acétal cycliques à des atomes d'oxygène de groupes hydroxyle adjacents, formés de préférence par la réaction d'aldéhyde substitué par N-(alk)acrylamino, habituellement sous la forme du dialkyl acétal, de préférence du N-acrylaminoacétaldéhyde diméthyl acétal.

8. Composition selon l'une quelconque des revendications précédentes, dans laquelle le polymère a une charge anionique d'au moins 1,0 meq g⁻¹.

9. Composition selon l'une quelconque des revendications précédentes, dans laquelle l'anthracycline est un composé de la formule générale II

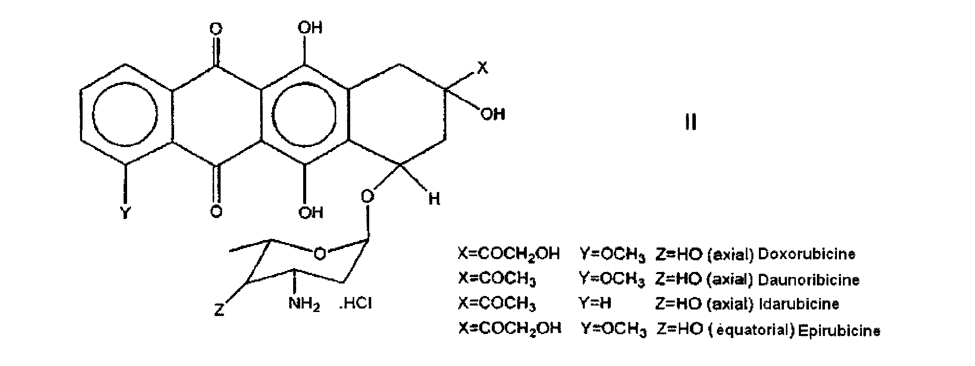

10. Composition selon la revendication 9, dans laquelle l'anthracycline est la doxorubicine.

11. Composition selon l'une quelconque des revendications précédentes, dans laquelle les particules sont des microsphères.

12. Composition selon l'une quelconque des revendications précédentes, dans laquelle les particules sont gonflées avec un liquide aqueux et suspendues dans celui-ci.

13. Composition selon la revendication 12, comprenant en outre un agent d'imagerie.

14. Composition selon la revendication 13, dans laquelle l'agent d'imagerie est un agent radio-opaque.

15. Composition selon l'une quelconque des revendications 1 à 11, dans laquelle les particules se présentent sous la forme d'une suspension épaisse pouvant être pompée comprenant des particules gonflées dans un liquide aqueux, sensiblement dépourvue de liquide extraparticulaire.

16. Composition selon la revendication 15, qui est chargée dans un récipient de stockage, de préférence une seringue, et est stérile.

17. Composition selon l'une quelconque des revendications 1 à 11, qui est sensiblement sèche.

18. Utilisation d'une anthracycline dans la fabrication d'une composition selon l'une quelconque des revendications précédentes pour une utilisation dans le traitement par embolothérapie d'une tumeur solide.

19. Procédé de production d'une composition selon l'une quelconque des revendications 1 à 17, dans laquelle des particules du polymère gonflant dans l'eau et insoluble dans l'eau sont mises en contact avec une solution de l'anthracycline en présence d'eau, moyennant quoi l'anthracycline est absorbée dans la matrice du polymère.

20. Procédé selon la revendication 19, dans lequel la mise en contact est réalisée par une mise en suspension des particules de polymère dans une solution aqueuse de l'anthracycline.

21. Procédé selon la revendication 20, dans lequel les particules de polymère avec l'anthracycline absorbée dans la matrice sont récupérées à partir de la suspension et séchées.

22. Procédé selon la revendication 21, dans lequel les particules sont séchées par lyophilisation.

23. Procédé selon la revendication 20, dans lequel les particules du polymère gonflé sont séparées du liquide surnageant et sont transférées alors qu'elles sont encore gonflées par le liquide de gonflement dans un récipient de stockage, de préférence une seringue, puis stérilisées et stockées dans le récipient.

24. Utilisation selon la revendication 18, dans laquelle la matrice polymère est formée par copolymérisation d'un macromère de poly(alcool vinylique) ayant au moins 2 groupes pendants éthyléniquement insaturés par molécule et un monomère anionique éthyléniquement insaturé.
